# FASCICULE DE BREVET EUROPEEN

(11) **EP 3 236 930 B1**
(45) Date de publication et mention de la délivrance du brevet: **05.02.2020**
(21) Numéro de dépôt: 15807929.3
(22) Date de dépôt: 10.12.2015
(51) Int. Cl.: A61K 8/81, A61Q 1/02, A61Q 1/04, A61K 8/31, A61K 8/04, A61Q 1/10, A61K 8/891

(54) **DISPERSION DE PARTICULES DE POLYMÈRE DANS UN MILIEU NON AQUEUX ET UTILISATION EN COSMÉTIQUE**
DISPERSION VON POLYMERPARTIKELN IN EINEM NICHTWÄSSRIGEN MEDIUM UND KOSMETISCHE VERWENDUNG DAVON
DISPERSION OF POLYMER PARTICLES IN A NON-AQUEOUS MEDIUM AND COSMETIC USE THEREOF

(30) Priorité: 22.12.2014 FR 1463082
(43) Date de publication de la demande: 01.11.2017
(73) Titulaire: L'Oréal, 75008 Paris (FR)
(72) Inventeur: PORTAL, Julien, 93320 Les Pavillons sous Bois (FR); SCHULTZE, Xavier, 93601 Aulnay-sous-Bois (FR); TAUPIN, Simon, 92160 Anthony (FR)
(74) Mandataire: L'Oreal
(86) Numéro de dépôt international: PCT/EP2015/079265
(87) Numéro de publication internationale: WO 2016/102191

(56) Documents cités:
- EP-A1- 0 749 747
- FR-A1- 2 880 267
- FR-A1- 2 972 630
- FR-A1- 2 972 631
- US-A1- 2011 243 864
- US-B1- 6 630 133
- "Rouge d'Armani Lasting Satin Lip Color", GNPD; MINTEL, mars 2011 (2011-03), - mars 2011 (2011-03), XP002742991,

## Description

La présente invention concerne une dispersion de particules de polymère dispersées dans un milieu non aqueux, ainsi qu'une composition cosmétique comprenant une telle dispersion.

Il est connu d'utiliser en cosmétique des dispersions de particules de polymère, dans des milieux organiques tels que des huiles hydrocarbonées comme dess hydrocarbures. Les polymères sont notamment utilisés comme agent filmogène dans des produits de maquillage tels que des mascaras, des eye-liners, des ombres à paupières ou des rouges à lèvres.

Le document EP-A-749747 décrit dans les exemples des dispersions dans des huiles hydrocarbonées (huile de parrafine, isododécane) de polymères acryliques stabilisés avec des copolymères dibloc polystyrène/copoly(éthylène-propylène). Toutefois, lorsque la teneur en matière sèche (polymère + stabilisant) dépasse les 25 % en poids, la dispersion devient alors trop visqueuse, engendrant ainsi des difficultés de formulation dans les produits cosmétiques en raison d'une modification importante de la viscosité de la composition finale de ces produits. En outre, le film obtenu après application de la dispersion sur la peau est peu brillant. Le document WO-A-2010/046229 décrit des dispersions dans l'isododécane de polymères acryliques stabilisés avec des polymères stabilisants séquencés, notamment tribloc, de monomères acryliques. Dans les exemples, selon l'exemple 1A, le polymère stabilisant est préparé par polymérisation radicalaire contrôlée par transfert de chaine réversible. Cette méthode de polymérisation est difficile à réaliser au stade industriel car elle nécessite un nombre important d'étapes intermédiaires de purification pour obtenir la dispersion finale de polymère.

Par ailleurs, les dispersions de polymères acryliques dans l'isododécane peuvent présenter des problèmes de compatibilité avec des huiles siliconées engendrant un déphasage de la dispersion.

Un besoin existe donc de disposer de dispersion stable de polymère acrylique stabilisé en milieu non aqueux comprenant une huile hydrocarbonée, facile à fabriquer industriellement, et permettant d'obtenir un film présentant de bonnes propriétés cosmétiques, notamment une bonne brillance, et étant également compatible avec les huiles siliconées.

La demanderesse a découvert que de nouvelles dispersions de particules de polymère de (méth)acrylate d'alkyle en C₁-C₄ stabilisées par un agent stabilisant particulier à base de polymère d'acrylate d'alkyle en C₈-C₂₂, le polymère et/ou le stabilisant comprenant un macromonomère siliconé particulier, dans une huile hydrocarbonée présentent de bonne propriété de stabilité, notamment après un stockage de 7 jours à la température ambiante (25 °C), sont faciles à fabriquer industriellement sans utiliser un grand nombre d'étapes de synthèse et permettent également d'obtenir un film après application sur un support présentant de bonnes propriétés cosmétiques en particulier une bonne brillance et une bonne résistance aux huiles. Ces dispersions présentent également une bonne compatibilité avec les huiles siliconées, en particulier lorsque le milieu de dispersion comprend jusqu'à 25 % en poids d'huile siliconée, par rapport au poids total d'huiles présentent dans la dispersion.

Un objet de la présente invention est donc une dispersion de particules d'au moins un polymère stabilisées par un agent stabilisant dans un milieu non aqueux contenant au moins une huile hydrocarbonée, le polymère des particules étant un polymère de (méth)acrylate d'alkyle en C₁-C₄ et éventuellement d'un macromonomère siliconé (I) tel que défini ci après ; l'agent stabilisant étant un polymère comprenant de 50 à 100 % en poids d'acrylate d'alkyle en C₈-C₂₂, de 0 à 50 % en poids de (méth)acrylate d'alkyle en C₁-C₄ et éventuellement de macronomère siliconé (I) tel que défini ci-après, par rapport au poids total du stabilisant, le stabilisant et/ou le polymère des particules comprenant au moins le macromonomère siliconé (I), et :
(i) lorsque le stabilisant comprend le macromonomère siliconé (I), le macromonomère est présent dans le stabilisant en une teneur inférieure ou égale à 5,5 % en poids, par rapport au poids total de l'ensemble stabilisant + polymère des particules ; le polymère des particules comprenant éventuellement ledit macromonomère siliconé (I) en une teneur inférieure ou égale à 28 % en poids, par rapport au poids total de l'ensemble stabilisant + polymère des particules ;
(ii) lorsque le polymère des particules comprend le macromonomère siliconé (I) et que le stabilisant ne comprend pas de macromonomère siliconé (I), le macromonomère est présent en une teneur inférieure ou égale à 18 % en poids, par rapport au poids total de l'ensemble stabilisant + polymère des particules.

La présence du macromonomère siliconé (I) dans le stabilisant et/ou le polymère des particules permet d'obtenir une dispersion de polymère stable, notamment après un stockage de 7 jours à la température ambiante (25 °C).

Un autre objet de l'invention est une composition comprenant, dans un milieu physiologiquement acceptable, une dispersion de particules de polymère telle que définie précédemment.

L'invention a également pour objet un procédé cosmétique non thérapeutique de traitement des matières kératiniques, comprenant l'application sur les matières kératiniques d'une composition telle que définie précédemment. Le procédé de traitement est en particulier un procédé de soin ou de maquillage des matières kératiniques.

Les dispersions selon l'invention sont donc constituées de particules, généralement sphériques, d'au moins un polymère dans un milieu non aqueux.

Le polymère des particules est un polymère de (méth)acrylate d'alkyle en C₁-C₄. Les monomères de (méth)acrylate d'alkyle en C₁-C₄ peuvent être choisis parmi le (méth)acrylate de méthyle, le (méth)acrylate d'éthyle, le (méth)acrylate de n-propyle, le (méth)acrylate d'isopropyle, le (méth)acrylate de n-butyle, le (méth)acrylate de tertio-butyle.

Avantageusement, on utilise un monomère d'acrylate d'alkyle en C₁-C₄. Préférentiellement, le polymère des particules est un polymère d'acrylate de méthyle et/ou, d'acrylate d'éthyle.

Le polymère des particules et/ou l'agent stabilisant peut comprendre un macromonomère polydiméthylsiloxane à groupement terminal mono(macryloyloxy ou monométhacryloyloxy de formule (I) (appelé par la suite macromonomère siliconé) suivante : 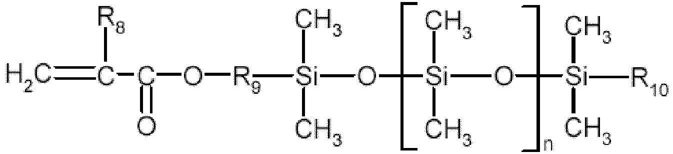 dans laquelle :
- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone, de préférence ayant de 2 à 4 atomes de carbone, et contenant éventuellement une ou deux liaisons éther -O- ; de préférence un groupe éthylène, propylène ou butylène;
- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

On peut en particulier utiliser les monométhacryloyloxypropyl polydiméthylsiloxanes tels que ceux commercialisés sous les dénominations MCR-M07, MCR-M17, MCR-M11, MCR-M22 par Gelest Inc ou X-22-2475, X-22-2426, X-22-174DX par Shin Etsu.

Le polymère des particules peut comprendre en outre un monomère acide à insaturation éthylénique ou de leur anhydride, notamment choisi parmi les monomères acides à insaturation éthylénique comprenant au moins une fonction acide carboxylique, phosphorique ou sulfonique, tel que l'acide crotonique, l'acide itaconique, l'acide fumarique, l'acide maléïque, l'anhydride maléïque, l'acide styrènesulfonique, l'acide vinylbenzoïque, l'acide vinylphosphorique, l'acide acrylique, l'acide méthacrylique, l'acide acrylamidopropanesulfonique, l'acide acrylamidoglycolique, et leurs sels.

De préférence, le monomère acide à insaturation éthylénique est choisi parmi l'acide (méth)acrylique, l'acide maléique et l'anhydride maléique.

Les sels peuvent être choisis parmi les sels de métal alcalin, par exemple sodium, potassium ; les sels de métal alcalino-terreux, par exemple calcium, magnésium, strontium, les sels métalliques, par exemple zinc, aluminium, manganèse, cuivre ; les sels d'ammonium de formule NH₄⁺ ; les sels d'ammonium quaternaires ; les sels d'amines organiques, comme par exemple les sels de méthylamine, de diméthylamine, de triméthylamine, de triéthylamine, d'éthylamine, de 2-hydroxyéthylamine, de bis-(2-hydroxyéthyl)amine, de la tri-(2-hydroxyéthyl)amine ; les sels de lysine, d'arginine.

Le polymère des particules peut ainsi comprendre, ou consiste essentiellement en, de 62 à 100 % en poids, de (méth)acrylate d'alkyle en C₁-C₄
et de 0 à 38 % en poids du macromonomère siliconé (I)
et de 0 à 20 % en poids de monomère acide à insaturation éthylénique, par rapport au poids total du polymère.

Selon un premier mode de réalisation de l'invention, le polymère consiste essentiellement en un polymère d'un ou plusieurs monomères de (méth)acrylate d'alkyle en C₁-C₄.

Selon un deuxième mode de réalisation de l'invention, le polymère consiste essentiellement en un copolymère de (méth)acrylate C₁-C₄ et d'acide (méth)acrylique ou d'anhydride maléique.

Selon un troisième mode de réalisation, le polymère consiste essentiellement en un copolymère de (méth)acrylate C₁-C₄ et de macromonomère siliconé (I). Selon un quatrième mode de réalisation de l'invention, le polymère consiste essentiellement en un copolymère de (méth)acrylate C₁-C₄ , d'acide (méth)acrylique ou d'anhydride maléique et de macromonomère siliconé (I).

Le polymère des particules peut être choisi parmi :
les homopolymères d'acrylate de méthyle
les copolymères acrylate de méthyle/macromonomère siliconé (I)
les homopolymères d'acrylate d'éthyle
les copolymères acrylate d'éthyle/macromonomère siliconé (I)
les copolymères acrylate de méthyle/acrylate d'éthyle
les copolymères acrylate de méthyle/acrylate d'éthyle/macromonomère siliconé (I)
les copolymères acrylate de méthyle/acrylate d'éthyle/acide acrylique
les copolymères acrylate de méthyle/acrylate d'éthyle/acide acrylique/macromonomère siliconé (I)
les copolymères acrylate de méthyle/acrylate d'éthyle/anhydride maléique
les copolymères acrylate de méthyle/acrylate d'éthyle/anhydride maléique/macromonomère siliconé (I)
les copolymères acrylate de méthyle/acide acrylique
les copolymères acrylate de méthyle/acide acrylique/macromonomère siliconé (I)
les copolymères acrylate d'éthyle/acide acrylique
les copolymères acrylate d'éthyle/acide acrylique/macromonomère siliconé (I)
les copolymères acrylate de méthyle/anhydride maléique
les copolymères acrylate de méthyle/anhydride maléique/macromonomère siliconé (I)
les copolymères acrylate d'éthyle/anhydride maléique/macromonomère siliconé (I)
les copolymères acrylate d'éthyle/anhydride maléique ;
et de préférence parmi :
   les copolymères acrylate de méthyle/acrylate
   les copolymères acrylate de méthyle/acrylate d'éthyle/macromonomère siliconé (I)
   les copolymères acrylate de méthyle/acrylate d'éthyle/acide acrylique.

Avantageusement, le polymère des particles est un polymère non réticulé.

Le polymère des particules de la dispersion a de préférence un poids moléculaire moyen en nombre allant de 2000 à 10000000, de préférence allant de 150 000 à 500 000.

Le polymère des particules peut être présent dans la dispersion en une teneur allant de 20 % à 60 % en poids, par rapport au poids total de la dispersion.

L'agent stabilisant est un polymère comprenant de 50 à 100 % en poids, de préférence de 60 à 95 % en poids, d'acrylate d'alkyle en C₈-C₂₂, de 0 à 50 % en poids, de préférence de 5 à 40 % en poids, de (méth)acrylate d'alkyle en C₁-C₄ et éventuellement le macronomère siliconé (I) tel que décrit précédemment. L'agent stabilisant est de préférence un polymère statistique.

L'acrylate d'alkyle en C₈-C₂₂ peut comprendre un groupe alkyle linéaire, ramifié ou cyclique comme par exemple un groupe 2-éthyle hexyle, isobornyle, lauryle, béhényle ou stéaryle.

De préférence, on utilise l'acrylate d'isobornyle et l'acrylate de 2-éthylhexyle. Préférentiellement, on utilise d'acrylate d'isobornyle.

Avantageusement, l'agent stabilisant est choisi parmi :
les homopolymères d'acrylate de 2-éthyl hexyle
les homopolymères d'acrylate d'isobornyle
les copolymères d'acrylate d'isobornyle/acrylate de méthyle
les copolymères d'acrylate 2-éthyl hexyle /acrylate de méthyle
les copolymères d'acrylate d'isobornyle/acrylate de méthyle/acrylate d'éthyle
les copolymères d'acrylate de 2-éthyl hexyle/acrylate de méthyle/acrylate d'éthyle
les copolymères d'acrylate d'isobornyle/acrylate de méthyle/acrylate d'éthyle/ macromonomère siliconé (I)
les copolymères d'acrylate de 2-éthylhexyle/acrylate de méthyle/acrylate d'éthyle/ macromonomère siliconé (I).

Le polymère stabilisant a de préférence un poids moléculaire moyen en nombre allant de 10 000 à 400 000 , de préférence allant de 20 000 à 200 000.

Le stabilisant est en contact avec la surface des particules de polymère et permet ainsi de stabiliser ces particules en surface pour le maintien de ces particles en dispersion dans le milieu non aqueux de la dispersion. Ainsi, les particules de polymère sont stabilisées en surface par l'agent stabilisant. Le stabilisant est un polymère distinct du polymère des particules : le stabilisant ne forme pas de liaison covalente avec le polymère des particules.

Selon un premier mode de réalisation selon l'invention, le macromonomère silicone (I) est présent dans le stabilisant de la dispersion ; il n'est pas présent dans le polymère des particules.

Le macromonomère siliconé (I) est présent dans le stabilisant en une teneur inférieure ou égale à 5,5 % en poids, notamment allant de 0,1 à 5,5 % en poids, par rapport au poids total de l'ensemble stabilisant + polymère des particules. En particulier, le macromonomère siliconé (I) peut être présent dans le stabilisant en une teneur allant de 0,1 à 35 % en poids, par rapport au poids total du stabilisant. Avantageusement, l'ensemble stabilisant + polymère des particules présents dans la dispersion comprend de 5 à 50 % en poids d'acrylate d'alkyle en C₈-C₂₂ polymérisé, de 44,5 à 89,5 % en poids de (méth)acrylate d'alkyle en C₁-C₄ polymérisé et de 0,1 à 5,5 % en poids de macromonomère siliconé (I), par rapport au poids total de l'ensemble stabilisant + polymère des particules. Préférentiellement, l'ensemble stabilisant + polymère des particules présent dans la dispersion comprend de 6 à 30 % en poids d'acrylate d'alkyle en C₈-C₂₂ polymérisé et de 64,5 à 93,9 % en poids de (méth)acrylate d'alkyle en C₁-C₄ polymérisé et de 0,1 à 5,5 % en poids de macromonomère siliconé (I), par rapport au poids total de l'ensemble stabilisant + polymère des particules.

Selon un deuxième mode de réalisation selon l'invention, le macromonomère silicone (I) est présent dans le stabilisant de la dispersion et dans le polymère des particules.

Le macromonomère siliconé (I) est présent dans le stabilisant en une teneur inférieure ou égale à 5,5 % en poids, notamment allant de 0,1 à 5,5 % en poids, par rapport au poids total de l'ensemble stabilisant + polymère des particules ; et il est présent dans le polymère des particules en une teneur inférieure ou égale à 28 % en poids, notamment allant de 0,1 à 28 % en poids, par rapport au poids total de l'ensemble stabilisant + polymère des particules.

En particulier, le macromonomère siliconé (I) peut être présent dans le stabilisant en une teneur allant de 0,1 à 35 % en poids, par rapport au poids total du stabilisant ; et il peut être présent dans le polymère des particules en une teneur allant de 0,1 à 38 % en poids, par rapport au poids total du polymère des particules. Avantageusement, l'ensemble stabilisant + polymère des particules présents dans la dispersion comprend de 5 à 50 % en poids d'acrylate d'alkyle en C₈-C₂₂ polymérisé, de 16,5 à 94,9 % en poids de (méth)acrylate d'alkyle en C₁-C₄ polymérisé et de 0,1 à 33,5 % en poids de macromonomère siliconé (I), par rapport au poids total de l'ensemble stabilisant + polymère des particules. Préférentiellement, l'ensemble stabilisant + polymère des particules présent dans la dispersion comprend de 6 à 30 % en poids d'acrylate d'alkyle en C₈-C₂₂ polymérisé et de 36,5 à 93,9 % en poids de (méth)acrylate d'alkyle en C₁-C₄ polymérisé et de 0,1 à 33,5 % en poids de macromonomère siliconé (I), par rapport au poids total de l'ensemble stabilisant + polymère des particules.

Selon un troisième mode de réalisation selon l'invention, le macromonomère silicone (I) est présent dans le polymère des particules de la dispersion ; il n'est pas présent dans le stabilisant.

Le macromonomère siliconé (I) est présent dans le polymère des particules en une teneur inférieure ou égale à 18 % en poids, notamment allant de 0,1 à 18 % en poids, par rapport au poids total de l'ensemble stabilisant + polymère des particules. Avantageusement, le macromonomère siliconé (I) est présent en une teneur allant de 0,1 à 25 % en poids, par rapport au poids total du polymère des particules.

Avantageusement, l'ensemble stabilisant + polymère des particules présents dans la dispersion comprend de 5 à 50 % en poids d'acrylate d'alkyle en C₈-C₂₂ polymérisé , de 32 à 94,9 % en poids de (méth)acrylate d'alkyle en C₁-C₄ polymérisé et de 0,1 à 18 % en poids de macromonomère siliconé (I), par rapport au poids total de l'ensemble stabilisant + polymère des particules.

Préférentiellement, l'ensemble stabilisant + polymère des particules présent dans la dispersion comprend de 6 à 30 % en poids d'acrylate d'alkyle en C₈-C₂₂ polymérisé et de 52 à 93,9 % en poids de (méth)acrylate d'alkyle en C₁-C₄ polymérisé et de 0,1 à 18 % en poids de macromonomère siliconé (I), par rapport au poids total de l'ensemble stabilisant + polymère des particules.

Le milieu huileux de la dispersion de polymère comprend une huile hydrocarbonée.

L'huile hydrocarbonée est une huile liquide à température ambiante (25 °C).

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

L'huile hydrocarbonée peut être choisie parmi :
les huiles hydrocarbonées ayant de 8 à 16 atomes de carbone, et notamment :
- les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls,
- les alcanes linéaires, par exemple tels que le n-dodécane (C12) et le n-tétradécane (C14) vendus par Sasol respectivement sous les références PARAFOL 12-97 et PARAFOL 14-97, ainsi que leurs mélanges, le mélange undécane-tridécane, les mélanges de n-undécane (C11) et de n-tridécane (C13) obtenus aux exemples 1 et 2 de la demande WO2008/155059 de la Société Cognis, et leurs mélanges.

les esters à chaîne courte (ayant de 3 à 8 atomes de carbone au total) tels que l'acétate d'éthyle, l'acétate de méthyle, l'acétate de propyle, l'acétate de n-butyle
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane , les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol.

Avantageusement, l'huile hydrocarbonée est apolaire (donc formée uniquement d'atomes de carbone et d'hydrogène).

L'huile hydrocarbonée est de préférence choisie parmi les huiles hydrocarbonées ayant de 8 à 16 atomes de carbone, en particulier les huiles apolaire, décrites précédemment.

Préférentiellement, l'huile hydrocarbonée est l'isododécane.

Les particules de polymère de la dispersion ont de préférence une taille moyenne, notamment en nombre, allant de 50 à 500 nm, notamment allant de 75 à 400 nm, et mieux allant de 100 à 250 nm.

D'une manière générale, la dispersion selon l'invention peut être préparée de la manière suivante, donnée à titre d'exemple.

La polymérisation peut être effectuée en dispersion, c'est-à-dire par précipitation du polymère en cours de formation, avec protection des particules formées avec un stabilisant.

Dans une première étape, on prépare le polymère stabilisant en mélangeant le ou les monomères constituant le polymère stabilisant, avec un amorceur radicalaire, dans un solvant appelé solvant de synthèse, et en polymérisant ces monomères. Dans une deuxième étape, on ajoute au polymère stabilisant formé le ou les monomères constituant le polymère des particules et on effectue la polymérisation de ces monomères ajoutés en présence de l'amorceur radicalaire.

Lorsque le milieu non aqueux est une huile hydrocarbonée non volatile, on peut effectuer la polymérisation dans un solvant organique apolaire (solvant de synthèse) puis ajouter l'huile hydrocarbonée non volatile (qui doit être miscible avec ledit solvant de synthèse) et distiller sélectivement le solvant de synthèse.

On choisit donc un solvant de synthèse tel que les monomères du polymère stabilisant, et l'amorceur radicalaire, y sont solubles, et les particules de polymère obtenu y sont insolubles afin qu'elles y précipitent lors de leur formation.

En particulier, on peut choisir le solvant de synthèse parmi les alcanes tels que l'heptane ou le cyclohexane.

Lorsque le milieu non aqueux est une huile hydrocarbonée volatile, on peut directement effectuer la polymérisation dans ladite huile qui joue donc également le rôle de solvant de synthèse. Les monomères doivent également y être solubles, ainsi que l'amorceur radicalaire, et le polymère des particules obtenu doit y être insoluble.

Les monomères sont de préférence présents dans le solvant de synthèse, avant polymérisation, à raison de 5-20% en poids. La totalité des monomères peut être présente dans le solvant avant le début de la réaction, ou une partie des monomères peut être ajoutée au fur et à mesure de l'évolution de la réaction de polymérisation.

L'amorceur radicalaire peut être notamment l'azo-bis-isobutyronitrile ou le tertiobutylperoxy-2-éthyl hexanoate.

La polymérisation peut être effectuée à une température allant de 70 à 110 °C.

Les particules de polymère sont stabilisées en surface, lorsqu'elles se forment lors de la polymérisation, grâce à l'agent stabilisant.

La stabilisation peut être effectuée par tout moyen connu, et en particulier par ajout direct de l'agent stabilisant, lors de la polymérisation.

Le stabilisant est de préférence également présent dans le mélange avant polymérisation des monomères du polymère des particules. Toutefois, il est également possible de l'ajouter en continu, notamment lorsque l'on ajoute également en continu les monomères du polymère des particules.

On peut utiliser de 10 à 30 % en poids de stabilisant par rapport au poids total de monomères mis en oeuvre (stabilisant + polymère des particules), et de préférence de 15 à 25 % en poids.

La dispersion de polymère obtenue selon l'invention peut être utilisée dans une composition comprenant un milieu physiologiquement acceptable, en particulier dans une composition cosmétique.

Par milieu physiologiquement acceptable, on entend un milieu compatible avec les matières kératiniques d'êtres humains, comme par exemple la peau, les lèvres, les ongles, les ciles, les sourcils, les cheveux.

Par composition cosmétique, on entend une composition compatible avec les matières kératiniques, qui présente une couleur, une odeur et un toucher agréables, et qui ne génère pas d'inconforts inacceptables (picotements, tiraillements, rougeurs), susceptibles d'en détourner le consommateur.

Par matières kératiniques, on entend la peau (corps, visage, contour des yeux, cuir chevelu), les cheveux, les cils, les sourcils, les poils, les ongles, les lèvres.

La composition selon l'invention peut comprendre un additif cosmétique choisi parmi l'eau, les parfums, les conservateurs, les charges, les matières colorantes, les filtres UV, les huiles, les cires, les tensioactifs, les hydratants, les vitamines, les céramides, les antioxydants, les agents anti radicaux libres, les polymères, les épaississants.

En particulier, la composition peut comprendre une huile siliconée, qui peut être choisie parmi les huiles de silicones linéaires ou cycliques volatiles, ayant notamment de 2 à 10 atomes de silicium, de préférence de 2 à 7 atomes de silicium, ces silicones comportant éventuellement des groupes alkyle ou alkoxy ayant de 1 à 10 atomes de carbone, comme par exemple l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane ;
les polydiméthylsiloxanes (PDMS) comportant des groupements alkyle ou alcoxy pendants et/ou en bouts de chaîne siliconée, groupements ayant chacun de 2 à 60 atomes de carbone, notamment les alkyl (C₂-C₆₀) dimethicones ; les alkyl(C₂-C₆₀) methicones ; les silicones phénylées comme les phényl triméthicones, les phényl diméthicones, les phényl triméthylsiloxy diphénylsiloxanes, les diphényl diméthicones, les triméthylsiloxyphenyl diméthicone, les diphényl méthyldiphényl trisiloxanes ;
et leurs mélanges.

L'huile siliconée peut être présente dans la composition selon l'invention en une teneur allant de 0,1 à 60 % en poids, par rapport au poids total de la composition, de préférence allant de 1 à 50 % en poids, et mieux allant de 1 à 40 % en poids.

La composition selon l'invention peut comprendre le polymère de la dispersion en une teneur allant de 1 à 50 % en poids, par rapport au poids total de la composition, et de préférence allant de 10 à 45 % en poids.

Avantageusement, la composition selon l'invention est une composition de maquillage, en particulier une composition de maquillage des lèvres, de mascara, d'eye-liner, de fard à paupières, de fond de teint.

Selon un mode de réalisation, la composition selon l'invention est une composition anhydre. On entend par composition anhydre une composition contenant moins de 2 % en poids d'eau, voire moins de 0,5 % d'eau, et notamment exempte d'eau. Le cas échéant, d'aussi faibles quantités d'eau peuvent notamment être amenées par des ingrédients de la composition qui peuvent en contenir des quantités résiduelles.

La composition selon l'invention peut être une composition aérosol contenant en plus un agent propulseur.

On peut utiliser comme agent propulseur des gaz liquéfiés comme le diméthyléther, les hydrocarbures chlorés et/ou fluorés tels que le trichlorofluorométhane, le dichlorodifluorométhane, le chlorodifluorométhane, le 1,1,1,2-tétrafluoroéthane, le chloropentafluoroéthane, le 1-chloro-1,1-difluoroéthane, le 1,1-difluoroéthane, ou des hydrocarbures volatils, tels que notamment les alcanes en C₃₋₅, comme le propane, l'isopropane, le n-butane, l'isobutane, le pentane, ou des gaz comprimés comme l'air, l'azote, le gaz carbonique, et leurs mélanges.

De préférence, on peut utliser le diméthyléther , le 1,1,1,2-tétrafluoroéthane, et les alcanes en C₃₋₅ et en particulier le propane, le n-butane, l'isobutane et leurs mélanges. Préférentiellement, on utilise l'isobutane.

La composition aérosol est généralement conditionnée, de façon connue, dans un dispositif aérosol comprenant un récipient et d'un moyen de distribution de la composition. Le moyen de distribution comprend généralement une valve de distribution commandée par une tête de distribution pouvant comporter une buse par laquelle la composition aérosol est vaporisée. L'homme du métier du domaine des aérosols est tout à fait apte à déterminer les caractéristiques du conditionnement approprié pour la distribution de la composition sous la forme de spray aérosol.

L'agent propulseur peut être présent dans la composition dans des proportions allant de 1 à 95 % en poids, de préférence de 1,5 à 50 % en poids, et mieux de 2 à 30 % en poids, par rapport au poids total de la composition.

Comme le montre les exemples, la dispersion huileuse selon l'invention présente une bonne compatibilité avec l'isobutane, notamment en présence d'huile de silicone polydiméthylsiloxane.

L'invention est illustrée plus en détail dans les exemples suivants. Les quantités sont indiquées en pourcentage pondéral.

### Evaluation des propriétés cosmétiques des dispersions huileuses :

On a déposé la dispersion huileuse à évaluer sur une carte de contraste (par exemple celle vendue sous la référence byko-charts par la société BYK-gardner) et le film déposé a été séché pendant 24 heures à la température ambiante (25 °C). Le film sec a une épaisseur d'environ 50 µm.

La brillance du film a été mesurée à l'aide d'un brillancemètre (trois angles Refo 3 / Refo 3D de chez Labomat) à un angle de 20°.

La résistance du film au corps gras été déterminée en déposant sur le film sec 3 gouttes d'huile d'olive sur la partie noire de la carte de contraste.Les gouttes ont été laissées en contact avec le film sec pendant respectivement 10 minutes, 30 minutes et 60 minutes et ensuite la goutte d'huile a été essuyée et on a observé l'aspect de la zone du film qui était en contact avec l'huile. Si le film a été endommagé par la goutte d'huile, le film de polymère est considéré comme non résistant à l'huile d'olive.

L'aspect collant du film de polymère a été évalué en touchant le film sec avec le doigt.

Tous les pourcentages de réactifs décrits dans les exemples sont des pourcentages pondéraux.

### Exemple 1

Dans une première étape, on a introduit dans un réacteur 120 g d'isododécane, 50 g d'acrylate d'isobornyle, 2 g d'acrylate de méthyle, 2 g d'acrylate de méthyle, 12,5 g de méthacrylate de polydiméthylsiloxane (X-22-2426 de chez Shin Etsu), 0,665 g de tertiobutylperoxy-2-éthylhexanoate (Trigonox 21S de chez Akzo). Le ratio massique acrylate d'isobornyle / acrylate de méthyle / acrylate d'éthyle / méthacrylate de PDMS est 75,2/3/3/18,8.

Le mélange a été chauffé à 90 °C , sous argon et sous agitation.

Après 2 heures de réaction, dans le pied de cuve du réacteur, on a ajouté 78 g d'isododécane et on a chauffé le mélange à 90 °C.

Dans une deuxième étape, on a introduit en une heure par une coulé un mélange de 91,5 g d'acrylate de méthyle, 91,5 g d'acrylate d'éthyle, 183 g d'isododécane et 1,83 g de Trigonox 21S, et on a laissé réagir pendant 7 heure. On a ensuite ajouté O,3 litre d'isododécane puis évaporé une partie de l'isododécane pour obtenir un extrait sec de 45 % en poids.

On a obtenu une dispersion de particules de copolymère acrylate de méthyle/acrylate d'éthyle (50/50 poids) stabilisées par un stabilisant copolymère statistique contenant 75,2 % en poids d'acrylate d'isobornyle, 3 % d'acrylate de méthyle, 3 % d'acrylate d'éthyle, 18,8 % de méthacrylate de PDMS, dans l'isododécane.

La dispersion huileuse contient au total (stabilisant + particules) 20 % d'acrylate d'isobornyle, 37,5 % d'acrylate de méthyle, 37,5 % d'acrylate d'éthyle et à 5 % de méthacrylate de PDMS.

Les particules du polymère de la dispersion ont une taille moyenne en nombre comprise environ entre 170 et 200 nm.

La dispersion est stable au stockage pendant 7 jours à la température ambiante (25 °C).

Le film obtenu avec la dispersion huileuse présente les propriétés suivantes :

| **Brillance à 20°** | **Résistance aux corps gras** | **Collant** |
|---|---|---|
| 73 | Résistant au corps gras | Non collant |

### Exemple 2

On a préparé une dispersion de particules de polymère dans l'isododécane, selon le mode opératoire de l'exemple 1, en utilisant :
Etape 1 : 50 g d'acrylate d'isobornyle, 2 g d'acrylate de méthyle, 2 g d'acrylate d'éthyle, 0,54 g de Trigonox 21S, 120 g d'isododécane ; puis ajout après réaction de 78 g d'isododécane
Etape 2 : 98 g d'acrylate de méthyle, 73 g d'acrylate d'éthyle, 25 g de méthacrylate de polydiméthylsiloxane (X-22-2426 de chez Shin Etsu), 1,96 g de Trigonox 21S, 196 g d'isododécane. Après réaction, ajout de 0,3 litre d'isododécane et évaporation pour obtenir un extrait sec de 40 % en poids.

On a obtenu une dispersion dans l'isododécane de particules de copolymère acrylate de méthyle/acrylate d'éthyle/acrylate de PDMS (50/37,2/12,8) stabilisées par un copolymère statistique acrylate d'isobornyle/ acrylate de méthyle/acrylate d'éthyle (92,6/3,7/3,7).

La dispersion huileuse contient au total (stabilisant + particules) 20 % d'acrylate d'isobornyle, 40 % d'acrylate de méthyle, 30 % d'acrylate d'éthyle et 10 % de méthacrylate de PDMS.

Les particules du polymère de la dispersion ont une taille moyenne en nombre comprise environ entre 170 et 200 nm.

La dispersion est stable au stockage pendant 7 jours à la température ambiante (25 °C).

Le film obtenu avec la dispersion huileuse présente les propriétés suivantes :

| **Brillance à 20°** | **Résistance aux corps gras** | **Collant** |
|---|---|---|
| 60 | Résistant au corps gras | Non collant |

### Exemple 3

On a préparé une dispersion de particules de polymère dans l'isododécane, selon le mode opératoire de l'exemple 1, en utilisant :
Etape 1 : 50 g d'acrylate d'isobornyle, 2 g d'acrylate de méthyle, 2 g d'acrylate d'éthyle, 12,5 g de méthacrylate de polydiméthylsiloxane (X-22-2426 de chez Shin Etsu), 0,665 g de Trigonox 21S, 120 g d'isododécane ; puis ajout après réaction de 78 g d'isododécane
Etape 2 : 91,5 g d'acrylate de méthyle, 79 g d'acrylate d'éthyle,12,5 g de méthacrylate de polydiméthylsiloxane (X-22-2426 de chez Shin Etsu), 1,83 g de Trigonox 21S, 183 g d'isododécane. Après réaction, ajout de 0,3 litre d'isododécane et évaporation pour obtenir un extrait sec de 42 % en poids.

On a obtenu une dispersion dans l'isododécane de particules de copolymère acrylate de méthyle/acrylate d'éthyle/acrylate de PDMS (50/43,2/6,8) stabilisées par un copolymère statistique acrylate d'isobornyle/ acrylate de méthyle/acrylate d'éthyle/méthacrylate de PDMS (75,2/3/3/18,8).

La dispersion huileuse contient au total (stabilisant + particules) 20 % d'acrylate d'isobornyle, 37,5 % d'acrylate de méthyle, 32,5 % d'acrylate d'éthyle et 10 % de méthacrylate de PDMS.

La dispersion est stable au stockage pendant 7 jours à la température ambiante (25 °C).

Le film obtenu avec la dispersion huileuse présente les propriétés suivantes :

| **Brillance à 20°** | **Résistance aux corps gras** | **Collant** |
|---|---|---|
| 74 | Résistant au corps gras | Non collant |

### Exemple 4

On a préparé une dispersion de particules de polymère dans l'isododécane, selon le mode opératoire de l'exemple 1, en utilisant :
Etape 1 : 50 g d'acrylate d'isobornyle, 2 g d'acrylate de méthyle, 2 g d'acrylate d'éthyle, 12,5 g de méthacrylate de polydiméthylsiloxane (X-22-2426 de chez Shin Etsu), 0,665 g de Trigonox 21S, 72 g d'isododécane et 48 g d'acétate d'éthyle ; puis ajout après réaction de 47 g d'isododécane et 31 g d'acétate d'éthyle
Etape 2 : 23 g d'acrylate de méthyle, 135,5 g d'acrylate d'éthyle, 1,835 g de Trigonox 21S, 183,5 g d'isododécane. Après réaction, ajout de 0,3 litre d'un mélange d'isododécane et d'acétate d'éthyle (60/40 poids) et évaporation totale de l'acétate d'éthyle et partielle de l'isododécane pour obtenir un extrait sec de 53 % en poids.

On a obtenu une dispersion dans l'isododécane de particules de copolymère acrylate de méthyle/acrylate d'éthyle/acide acrylique (12,5/73,9/13,6) stabilisées par un copolymère statistique acrylate d'isobornyle/ acrylate de méthyle/acrylate d'éthyle/méthacrylate de PDMS (75,2/3/3/18,8).

La dispersion huileuse contient au total (stabilisant + particules) 20 % d'acrylate d'isobornyle, 10 % d'acrylate de méthyle, 55 % d'acrylate d'éthyle, 10 % d'acide acrylique et 5 % de méthacrylate de PDMS.

La dispersion est stable au stockage pendant 7 jours à la température ambiante (25 °C).

Le film obtenu avec la dispersion huileuse présente les propriétés suivantes :

| **Brillance à 20°** | **Résistance aux corps gras** | **Collant** |
|---|---|---|
| 67 | Résistant au corps gras | Non collant |

### Exemple 5

On a préparé une dispersion de particules de polymère dans l'isododécane, selon le mode opératoire de l'exemple 1, en utilisant :
Etape 1 : 25 g d'acrylate de 2-éthylhexyle, 2 g d'acrylate de méthyle, 2 g d'acrylate d'éthyle, 12,5 g de méthacrylate de polydiméthylsiloxane (X-22-2426 de chez Shin Et-su), 0,415 g de Trigonox 21S, 120 g d'isododécane ; puis ajout après réaction de 78 g d'isododécane
Etape 2 : 104 g d'acrylate de méthyle, 104 g d'acrylate d'éthyle, 2,08 g de Trigonox 21S, 208 g d'isododécane. Après réaction, ajout de 0,3 litre d'isododécane et évaporation pour obtenir un extrait sec de 45 % en poids.

On a obtenu une dispersion dans l'isododécane de particules de copolymère acrylate de méthyle/acrylate d'éthyle (50/50) stabilisées par un copolymère statistique acrylate de 2-éyhylhexyle/ acrylate de méthyle/acrylate d'éthyle/méthacrylate de PDMS (60,3/4,8/4,8/30,1).

La dispersion huileuse contient au total (stabilisant + particules) 10 % d'acrylate de 2-éthylhexyle, 42,5 % d'acrylate de méthyle, 42,5 % d'acrylate d'éthyle et 5 % de méthacrylate de PDMS.

La dispersion est stable au stockage pendant 7 jours à la température ambiante (25 °C).

Le film obtenu avec la dispersion huileuse présente les propriétés suivantes :

| **Brillance à 20°** | **Résistance aux corps gras** | **Collant** |
|---|---|---|
| 51 | Résistant au corps gras | Collant |

### Exemple 6

On a préparé une dispersion de particules de polymère dans l'isododécane, selon le mode opératoire de l'exemple 1, en utilisant :
Etape 1 : 25 g d'acrylate de 2-éthylhexyle, 2 g d'acrylate de méthyle, 2 g d'acrylate d'éthyle, 0,29 g de Trigonox 21S, 120 g d'isododécane ; puis ajout après réaction de 78 g d'isododécane
Etape 2 : 110,5 g d'acrylate de méthyle, 85,5 g d'acrylate d'éthyle, 25 g de méthacrylate de polydiméthylsiloxane (X-22-2426 de chez Shin Etsu), 2,21 g de Trigonox 21S, 221 g d'isododécane. Après réaction, ajout de 0,3 litre d'isododécane et évaporation pour obtenir un extrait sec de 43 % en poids.

On a obtenu une dispersion dans l'isododécane de particules de copolymère acrylate de méthyle/acrylate d'éthyle/acrylate de PDMS (50/38,7/11,3) stabilisées par un copolymère statistique acrylate de 2-éthylhexyle/ acrylate de méthyle/acrylate d'éthyle (86,2/6,9/6,9).

La dispersion huileuse contient au total (stabilisant + particules) 10% d'acrylate de 2-éthylhexyle, 45 % d'acrylate de méthyle, 35 % d'acrylate d'éthyle et 10 % de méthacrylate de PDMS.

La dispersion est stable au stockage pendant 7 jours à la température ambiante (25 °C).

Le film obtenu avec la dispersion huileuse présente les propriétés suivantes :

| **Brillance à 20°** | **Résistance aux corps gras** | **Collant** |
|---|---|---|
| 63 | Résistant au corps gras | Collant |

### Exemple 7 :

On a préparé une dispersion de particules de polymère dans l'isododécane, selon le mode opératoire de l'exemple 1, en utilisant :
Etape 1 : 25 g d'acrylate de 2-éthylhexyle, 2 g d'acrylate de méthyle, 2 g d'acrylate d'éthyle, 12,5 g de méthacrylate de polydiméthylsiloxane (X-22-2426 de chez Shin Et-su), 0,415 g de Trigonox 21S, 120 g d'isododécane ; puis ajout après réaction de 78 g d'isododécane
Etape 2 : 104 g d'acrylate de méthyle, 91,5 g d'acrylate d'éthyle,12,5 g de méthacrylate de polydiméthylsiloxane (X-22-2426 de chez Shin Etsu), 2,08 g de Trigonox 21S, 208 g d'isododécane. Après réaction, ajout de 0,3 litre d'isododécane et évaporation pour obtenir un extrait sec de 46 % en poids.

On a obtenu une dispersion dans l'isododécane de particules de copolymère acrylate de méthyle/acrylate d'éthyle/acrylate de PDMS (50/44/6) stabilisées par un copolymère statistique acrylate de 2-éthylhexyle/ acrylate de méthyle/acrylate d'éthyle/méthacrylate de PDMS (60,2/4,8/4,8/3028).

La dispersion huileuse contient au total (stabilisant + particules) 10 % d'acrylate de 2-éthylhexyle, 42,5 % d'acrylate de méthyle, 37,5 % d'acrylate d'éthyle et 10 % de méthacrylate de PDMS.

La dispersion est stable au stockage pendant 7 jours à la température ambiante (25 °C).

Le film obtenu avec la dispersion huileuse présente les propriétés suivantes :

| **Brillance à 20°** | **Résistance aux corps gras** | **Collant** |
|---|---|---|
| 54 | Résistant au corps gras | Collant |

### Etude de la compatibilité avec des huiles siliconées :

On a évalué la compatibilité des dispersions de particules de polymères effectéues en ajoutant dans la dispersion 5 huiles de silicone différentes (silicone 1 à 5) et en observant si le mélange obtenu est stable ou non (mélange homogène ou hétérogène)
Silicone 1 : Polyphényltriméthylsiloxy diméthylsiloxane (Belsil® PDM 1000 de chez Wacker) (nom INCI : TRIMETHYLSILOXYPHENYL DIMETHICONE) Silicone 2 : cyclohexadiméthylsiloxane
Silicone 3: 3-octylheptaméthyl trisiloxane (DOW CORNING FZ-3196 de chez Dow Corning)
Silicone 4 : polydiméthylsiloxane 5 cst (XIAMETER PMX-200 SILICIONE FLUIDS 5CS de chez Dow Corning)
Silicone 5 : dodecamethylpentasiloxane

On a obtenu les résultats suivants :

| Exemple | Ratio HC/Si | S1 | S2 | S3 | S4 | S5 |
|---|---|---|---|---|---|---|
| 1 | 75/25 | + | + | + | + | + |
| | 50/50 | + | + | + | - | + |
| | 25/75 | - | + | + | - | - |
| 2 | 75/25 | + | - | + | - | - |
| | 50/50 | + | - | + | - | - |
| | 25/75 | - | - | + | - | - |
| 3 | 75/25 | + | + | + | + | + |
| | 50/50 | + | + | + | - | + |
| | 25/75 | + | - | + | - | - |
| 4 | 75/25 | + | + | + | + | + |
| | 50/50 | + | + | + | - | + |
| | 25/75 | - | + | + | - | - |
| 5 | 75/25 | + | + | + | + | + |
| | 50/50 | + | + | + | + | + |
| | 25/75 | + | + | + | + | + |
| 6 | 75/25 | + | + | + | + | + |
| | 50/50 | + | + | + | + | + |
| | 25/75 | + | + | + | + | + |
| 7 | 75/25 | + | + | + | + | + |
| | 50/50 | + | + | + | + | + |
| | 25/75 | + | + | + | + | + |

Ratio HC / Si : ratio pondéral huile hydrocarbonée (isosododécane)/huile siliconée présents dans la dispersion de particules de polymère.

Les résultats obtenus montrent que les dispersions des exemples 5 à 7 ont une bonne compatibilité avec les 5 huiles siliconées dans les 3 ratio d'huiles. Les dispersions des exemples 1, 3, 4 ont une bonne compatibilité avec les 5 huiles siliconée au ratio HC/Si = 75/25 et avec 4 huiles siliconées au ratio de 50/50 et avec 2 huiles siliconées au ratio 25/75. La dispersion de l'exemple 2 est bien compatible avec la silicone 3 (tous ratios) et avec la silicone 1 (ratios 25/75 et 50/50).

### Exemples 8 et 9 :

On a préparé, selon le mode opératoire de l'exemple 1, deux dispersions huileuses de copolymère acrylate d'isobornyle, acrylate de méthyle et un méthacrylate de polydiméthysiloxane dont la chaîne PDMS a un poids moléculaire différent.

Toutes les dispersions comprennent au total (stabilisant + particules) 20 % d'acrylate d'isobornyle, 78 % d'acrylate de méthyle et 2 % de méthacrylate de PDMS.

On a évalué la compatibilité de ces 2 dispersions avec les 5 huiles de silicone.

### Exemple 8 :

Etape 1 : 50 g d'acrylate d'isobornyle, 5 g d'acrylate de méthyle, 4 g de méthacrylate de polydiméthylsiloxane (X-22-2475 de chez Shin Etsu ayant une chaîne PDMS de poids moléculaire de 420 g/mole), 0,59 g de Trigonox 21, 120 g d'isododécane ; puis ajout après réaction de 68 g d'isododécane
Etape 2: 191 g d'acrylate de méthyle, 1,91 g de Trigonox 21S, 191 g d'isododécane. Après réaction, ajout de 300 g d'isododécane et évaporation pour obtenir un extrait sec de 44 % en poids.

On a obtenu une dispersion dans l'isododécane de particules de polyacrylate de méthyle stabilisées par un stabilisant polyacrylate d'isobornyle/acrylate de méthyle/acrylate de PDMS (84,7/8,5/6,8).

La dispersion huileuse contient au total (stabilisant + particules) 20 % d'acrylate d'isobornyle, 78 % d'acrylate de méthyle et 2 % de méthacrylate de PDMS.

La dispersion est stable au stockage pendant 7 jours à la température ambiante (25 °C). Le film obtenu est non collant.

### Exemple 9 :

Etape 1 : 50 g d'acrylate d'isobornyle, 5 g d'acrylate de méthyle, 4 g de méthacrylate de polydiméthylsiloxane (X-22-2426 de chez Shin Etsu ayant une chaîne PDMS de 12 000 g/mole), 0,59 g de Trigonox 21, 120 g d'isododécane ; puis ajout après réaction de 68 g d'isododécane
Etape 2: 191 g d'acrylate de méthyle, 1,91 g de Trigonox 21S, 191 g d'isododécane. Après réaction, ajout de 300 g d'isododécane et évaporation pour obtenir un extrait sec de 47 % en poids.

On a obtenu une dispersion dans l'isododécane de particules de polyacrylate de méthyle stabilisées par un stabilisant polyacrylate d'isobornyle/acrylate de méthyle/acrylate de PDMS (84,7/8,5/6,8).

La dispersion est stable au stockage pendant 7 jours à la température ambiante (25 °C). Le film obtenu est non collant.

On a obtenu les résultats de compatibilité suivants :

| Exemple | Ratio HC/Si | S1 | S2 | S3 | S4 | S5 |
|---|---|---|---|---|---|---|
| 8 | 75/25 | + | + | + | - | + |
| | 50/50 | + | - | - | - | - |
| | 25/75 | - | - | - | - | - |
| 9 | 75/25 | + | + | + | - | + |
| | 50/50 | + | + | + | - | + |
| | 25/75 | - | + | - | - | - |

Les résultats obtenus montrent que la dispersion de l'exemple 9 a une compatibilité avec les huiles siliconées plus importante que celle de l'exemple 8. Cette dernière est compatible avec 4 huiles siliconées au ratio de 75/25.

### Etude sur le ratio de macromonomère siliconé dans le stabilisant :

### Exemples 1, 10, 11 (invention) et 12 à 14 (hors invention)

On a préparé, selon le mode opératoire de l'exemple 1, 5 dispersions huileuses de copolymère acrylate d'isobornyle, acrylate de méthyle, acrylate d'éthyle et méthacrylate de polydiméthysiloxane (X-22-2426 de chez Shin Etsu ayant une chaîne PDMS de 12 000 g/mole) en faisant varier la teneur en macromonomère siliconé (teneur compensée sur celles des acrylates de méthyle et d'éthyle).

### Exemple 10 :

Etape 1 : 50 g d'acrylate d'isobornyle, 2 g d'acrylate de méthyle, 2 g d'acrylate d'éthyle, 5 g de méthacrylate de polydiméthylsiloxane (X-22-2426 de chez Shin Etsu), 0,59 g de Trigonox 21, 120 g d'isododécane ; puis ajout après réaction de 68 g d'isododécane
Etape 2 : 95,5 g d'acrylate de méthyle, 95,5 g d'acrylate d'éthyle, 1,91 g de Trigonox 21S, 191 g d'isododécane. Après réaction, ajout de 300 g d'isododécane et évaporation pour obtenir un extrait sec de 43 % en poids.

On a obtenu une dispersion dans l'isododécane de particules de polyacrylate de méthyle/acrylate d'éthyle (50/50) stabilisées par un stabilisant polyacrylate d'isobornyle/acrylate de méthyle/acrylate d'éthyle/acrylate de PDMS (84,7/3,4/3,4/8,5).

La dispersion huileuse contient au total (stabilisant + particules) 20 % d'acrylate d'isobornyle, 39 % d'acrylate de méthyle , 39 % d'acrylate d'éthyle et 2 % de méthacrylate de PDMS.

### Exemple 11 :

Etape 1 : 50 g d'acrylate d'isobornyle, 2 g d'acrylate de méthyle, 2 g d'acrylate d'éthyle, 10 g de méthacrylate de polydiméthylsiloxane (X-22-2426 de chez Shin Etsu), 0,64 g de Trigonox 21, 120 g d'isododécane ; puis ajout après réaction de 78 g d'isododécane
Etape 2 : 93 g d'acrylate de méthyle, 93 g d'acrylate d'éthyle, 1,86 g de Trigonox 21S, 186 g d'isododécane. Après réaction, ajout de 300 g d'isododécane et évaporation pour obtenir un extrait sec de 46 % en poids.

On a obtenu une dispersion dans l'isododécane de particules de polyacrylate de méthyle/acrylate d'éthyle (50/50) stabilisées par un stabilisant polyacrylate d'isobornyle/acrylate de méthyle/acrylate d'éthyle/acrylate de PDMS (78,2/3,1/3,1/15,6).

### Exemple 12 :

Etape 1 : 50 g d'acrylate d'isobornyle, 2 g d'acrylate de méthyle, 2 g d'acrylate d'éthyle, 15,2 g de méthacrylate de polydiméthylsiloxane (X-22-2426 de chez Shin Et-su), 0,7 g de Trigonox 21, 120 g d'isododécane ; puis ajout après réaction de 78 g d'isododécane
Etape 2 : 90 g d'acrylate de méthyle, 90 g d'acrylate d'éthyle, 1,8 g de Trigonox 21S, 180 g d'isododécane. Après réaction, ajout de 300 g d'isododécane et évaporation.

On a obtenu une dispersion instable dans l'isododécane de particules de polyacrylate de méthyle/acrylate d'éthyle (50/50) en présence de stabilisant polyacrylate d'isobornyle/acrylate de méthyle/acrylate d'éthyle/acrylate de PDMS (72,5/2,9/2,9/21,7).

La dispersion huileuse instable contient au total (stabilisant + particules) 20 % d'acrylate d'isobornyle, 37 % d'acrylate de méthyle , 37 % d'acrylate d'éthyle et 6 % de méthacrylate de PDMS.

### Exemple 13 :

Etape 1 : 50 g d'acrylate d'isobornyle, 2 g d'acrylate de méthyle, 2 g d'acrylate d'éthyle, 25,10 g de méthacrylate de polydiméthylsiloxane (X-22-2426 de chez Shin Etsu), 0,79 g de Trigonox 21, 120 g d'isododécane ; puis ajout après réaction de 78 g d'isododécane
Etape 2 : 85,5 g d'acrylate de méthyle, 85,5 g d'acrylate d'éthyle, 1,71 g de Trigonox 21S, 171 g d'isododécane. Après réaction, ajout de 300 g d'isododécane et évaporatio.

On a obtenu une dispersion instable dans l'isododécane de particules de polyacrylate de méthyle/acrylate d'éthyle (50/50) en présence de stabilisant polyacrylate d'isobornyle/acrylate de méthyle/acrylate d'éthyle/acrylate de PDMS (78,2/3,1/3,1/15,6).

La dispersion huileuse instable contient au total (stabilisant + particules) 20 % d'acrylate d'isobornyle, 35 % d'acrylate de méthyle , 35 % d'acrylate d'éthyle et 10 % de méthacrylate de PDMS.

### Exemple 14 :

Etape 1 : 50 g d'acrylate d'isobornyle, 2 g d'acrylate de méthyle, 2 g d'acrylate d'éthyle, 20 g de méthacrylate de polydiméthylsiloxane (X-22-2426 de chez Shin Etsu), 0,74 g de Trigonox 21, 120 g d'isododécane ; puis ajout après réaction de 78 g d'isododécane
Etape 2 : 88 g d'acrylate de méthyle, 88 g d'acrylate d'éthyle, 1,76 g de Trigonox 21S, 176 g d'isododécane. Après réaction, ajout de 300 g d'isododécane et évaporation.

On a obtenu une dispersion instable dans l'isododécane de particules de polyacrylate de méthyle/acrylate d'éthyle (50/50) en présence de stabilisant polyacrylate d'isobornyle/acrylate de méthyle/acrylate d'éthyle/acrylate de PDMS (78,2/3,1/3,1/15,6).

La dispersion huileuse instable contient au total (stabilisant + particules) 20 % d'acrylate d'isobornyle, 36 % d'acrylate de méthyle , 36 % d'acrylate d'éthyle et 8 % de méthacrylate de PDMS.

On a évalué la stabilité de chaque dispersion après 12 heures de stockage à la température ambiante. On a obtenu les résultats suivants :

| **Exemples** | **Monomères** | **Pourcentage massique des monomères** | **Solvant** | **Extrait sec (%)** | **Stabilité** |
|---|---|---|---|---|---|
| **10** | AI | 20 | Isododécane | 43.44 | oui |
| | AM | 39 | | | |
| | AE | 39 | | | |
| | MPDMS12K | 2 | | | |
| **11** | AI | 20 | Isododécane | 45.80 | oui |
| | AM | 38 | | | |
| | AE | 38 | | | |
| | MPDMS 12K | 4 | | | |
| **1** | AI | 20 | Isododécane | 42.6 | oui |
| | AM | 37.5 | | | |
| | AE | 37.5 | | | |
| | MPDMS12K | 5 | | | |
| **12** | AI | 20 | Isododécane | - | non |
| | AM | 37 | | | |
| | AE | 37 | | | |
| | MPDMS12K | 6 | | | |
| **13** | AI | 20 | Isododécane | - | non |
| | AM | 35 | | | |
| | AE | 35 | | | |
| | MPDMS 12K | 10 | | | |
| **14** | AI | 20 | Isododécane | - | non |
| | AM | 36 | | | |
| | AE | 36 | | | |
| | MPDMS12K | 8 | | | |

| | | | | | |
|---|---|---|---|---|---|
| *AI* = *Acrylate d'Isobornyle* - *AM* = *Acrylate de Méthyle* - *AE* = *Acrylate d'Ethyle - MPDM12k* = *Méthacrylate de PDMS* (X-22-2426 de chez Shin Etsu) | | | | | |

Les résultats obtenus montrent que lorsque le méthacrylate de PDMS est présent en une teneur supérieure ou égale à 6 % du poids total des monomères formant le stabilisant, les dispersions (exemples 12 à 14) ne sont pas stables. Les dispersions des exemples 1, 10, 11 selon l'invention sont stables.

### Etude sur le ratio de macromonomère siliconé dans le polymère des particules :

### Exemple 2 (invention) et 15 (hors invention) :

On a préparé, selon le mode opératoire de l'exemple 2 , une dispersion de copolymère acrylate d'isobornyle, acrylate de méthyle, acrylate d'éthyle et et méthacrylate de polydiméthysiloxane (X-22-2426 de chez Shin Etsu ayant une chaîne PDMS de 12 000 g/mole) en faisant varier la teneur en macromonomère siliconé (teneur compensée sur celles des acrylates de méthyle et d'éthyle).

### Exemple 15 :

Etape 1 : 50 g d'acrylate d'isobornyle, 2 g d'acrylate de méthyle, 2 g d'acrylate d'éthyle, 0,54 g de Trigonox 21S, 120 g d'isododécane ; puis ajout après réaction de 78 g d'isododécane
Etape 2 : 98 g d'acrylate de méthyle, 73 g d'acrylate d'éthyle, 25 g de méthacrylate de polydiméthylsiloxane (X-22-2426 de chez Shin Etsu), 1,96 g de Trigonox 21S, 196 g d'isododécane. Après réaction, ajout de 0,3 litre d'isododécane et évaporation.

On a obtenu une dispersion instable dans l'isododécane de particules de polyacrylate de méthyle/acrylate d'éthyle (50/50) en présence de stabilisant polyacrylate d'isobornyle/acrylate de méthyle/acrylate d'éthyle/acrylate de PDMS (67,6/2,7/2,7/20).

La dispersion huileuse instable contient au total (stabilisant + particules) 20 % d'acrylate d'isobornyle, 40 % d'acrylate de méthyle , 20 % d'acrylate d'éthyle et 20 % de méthacrylate de PDMS.

On a évalué la stabilité de chaque dispersion après 12 heures de stockage à la température ambiante. On a obtenu les résultats suivants :

| **Exemples** | **Monomères** | **Pourcentage massique des monomères** | **Solvant** | **Extrait sec (%)** | **Stabilité** |
|---|---|---|---|---|---|
| **2** | AI | 20 | Isododécane | 39.82 | oui |
| | AM | 40 | | | |
| | AE | 30 | | | |
| | MPDMS12K | 10 | | | |
| **15** | AI | 20 | Isododécane | -- | non |
| | AM | 40 | | | |
| | AE | 20 | | | |
| | MPDMS 12K | 20 | | | |

| | | | | | |
|---|---|---|---|---|---|
| *AI* = *Acrylate d'Isobornyle* - *AM* = *Acrylate de Méthyle* - *AE* = *Acrylate d'Ethyle - MPDMS12k = Méthacrylate de PDMS* (X-22-2426 de chez Shin Etsu) | | | | | |

Les résultats obtenus montrent que lorsque le macromonomère siliconé est présent dans le polymère des particules en une teneur de 20 % la dispersion n'est pas stable.

### Etude sur le ratio de macromonomère siliconé dans le stabilisant et dans le polymère des particules :

### Exemple 3, 16, 17 (invention) et 18 (hors invention) :

On a préparé, selon le mode opératoire de l'exemple 3 , 3 dispersions de copolymère acrylate d'isobornyle, acrylate de méthyle, acrylate d'éthyle et et méthacrylate de polydiméthysiloxane (X-22-2426 de che z Shin Etsu ayant une chaîne PDMS de 12 000 g/mole) en faisant varier la teneur en macromonomère siliconé (teneur compensée sur celles des acrylates de méthyle et d'éthyle) dans la stabilisant et dans le polymère des particules.

### Exemple 16 :

Etape 1 : 50 g d'acrylate d'isobornyle, 2 g d'acrylate de méthyle, 2 g d'acrylate d'éthyle, 12,5 g de méthacrylate de polydiméthylsiloxane (X-22-2426 de chez Shin Etsu), 0,665 g de Trigonox 21S, 120 g d'isododécane ; puis ajout après réaction de 78 g d'isododécane
Etape 2 : 91,5 g d'acrylate de méthyle, 66,5 g d'acrylate d'éthyle, 25 g de méthacrylate de polydiméthylsiloxane (X-22-2426 de chez Shin Etsu), 1,83 g de Trigonox 21S, 183 g d'isododécane. Après réaction, ajout de 300 g d'isododécane et évaporation pour obtenir un extrait sec de 44 % en poids.

On a obtenu une dispersion dans l'isododécane de particules de copolymère acrylate de méthyle/acrylate d'éthyle/acrylate de PDMS (50/36,3/13,7) stabilisées par un copolymère statistique acrylate d'isobornyle/ acrylate de méthyle/acrylate d'éthyle/méthacrylate de PDMS (75,2/3/3/18,8).

La dispersion huileuse contient au total (stabilisant + particules) 20 % d'acrylate d'isobornyle, 37,5 % d'acrylate de méthyle, 27,5 % d'acrylate d'éthyle et 25 % de méthacrylate de PDMS.

### Exemple 17 :

Etape 1 : 50 g d'acrylate d'isobornyle, 2 g d'acrylate de méthyle, 2 g d'acrylate d'éthyle, 12,5 g de méthacrylate de polydiméthylsiloxane (X-22-2426 de chez Shin Etsu), 0,665 g de Trigonox 21S, 120 g d'isododécane ; puis ajout après réaction de 78 g d'isododécane
Etape 2 : 91,5 g d'acrylate de méthyle, 41,5 g d'acrylate d'éthyle, 50 g de méthacrylate de polydiméthylsiloxane (X-22-2426 de chez Shin Etsu), 1,83 g de Trigonox 21S, 183 g d'isododécane. Après réaction, ajout de 300 g d'isododécane et évaporation pour obtenir un extrait sec de 42 % en poids.

On a obtenu une dispersion dans l'isododécane de particules de copolymère acrylate de méthyle/acrylate d'éthyle/acrylate de PDMS (50/22,7/27,3) stabilisées par un copolymère statistique acrylate d'isobornyle/ acrylate de méthyle/acrylate d'éthyle/méthacrylate de PDMS (75,2/3/3/18,8).

La dispersion huileuse contient au total (stabilisant + particules) 20 % d'acrylate d'isobornyle, 37,5 % d'acrylate de méthyle, 17,5 % d'acrylate d'éthyle et 25 % de méthacrylate de PDMS.

### Exemple 18 :

Etape 1 : 50 g d'acrylate d'isobornyle, 2 g d'acrylate de méthyle, 2 g d'acrylate d'éthyle, 25 g de méthacrylate de polydiméthylsiloxane (X-22-2426 de chez Shin Etsu), 0,79 g de Trigonox 21S, 120 g d'isododécane ; puis ajout après réaction de 78 g d'isododécane
Etape 2 : 85,2 g d'acrylate de méthyle, 60,2 g d'acrylate d'éthyle, 25 g de méthacrylate de polydiméthylsiloxane (X-22-2426 de chez Shin Etsu), 1,70 g de Trigonox 21S, 170 g d'isododécane. Après réaction, ajout de 300 g d'isododécane et évaporation.

On a obtenu une dispersion instable dans l'isododécane de particules de copolymère acrylate de méthyle/acrylate d'éthyle/acrylate de PDMS (50/35,3/14,7) en présence d'un copolymère statistique acrylate d'isobornyle/ acrylate de méthyle/acrylate d'éthyle/méthacrylate de PDMS (63,3/2,5/2,5/31,7).

La dispersion huileuse contient au total (stabilisant + particules) 20 % d'acrylate d'isobornyle, 35 % d'acrylate de méthyle, 25 % d'acrylate d'éthyle et 20 % de méthacrylate de PDMS.

On a évalué la stabilité de chaque dispersion après 12 heures de stockage à la température ambiante. On a obtenu les résultats suivants :

| **Exemples** | **Monomères** | **Pourcentage massique des monomères** | **Solvant** | **Extrait sec (%)** | **Stabilité** |
|---|---|---|---|---|---|
| **3** | AI | 20 | Isododécane | 45.02 | Oui |
| | AM | 37.5 | | | |
| | AE | 32.5 | | | |
| | MPDMS12K (stabilisant) | 5 | | | |
| | MPDMS12K (particules) | 5 | | | |
| **16** | AI | 20 | Isododécane | 44.10 | Oui |
| | AM | 37.5 | | | |
| | AE | 27.5 | | | |
| | MPDMS12K (stabilisant) | 5 | | | |
| | MPDMS12K (particules) | 10 | | | |
| **17** | AI | 20 | Isododécane | 38.94 | Oui |
| | AM | 37.5 | | | |
| | AE | 17.5 | | | |
| | MPDMS12K (stabilisant) | 5 | | | |
| | MPDMS12K (particules) | 20 | | | |
| **18** | AI | 20 | Isododécane | - | non |
| | AM | 35 | | | |
| | AE | 25 | | | |
| | MPDMS12K (stabilisant) | 10 | | | |
| | MPDMS12K (particules) | 10 | | | |

| | | | | | |
|---|---|---|---|---|---|
| *AI* = *Acrylate d'Isobornyle* - *AM* = *Acrylate de Méthyle* - *AE* = *Acrylate d'Ethyle - MPDMS12k* = *Méthacrylate de PDMS* (X-22-2426 de chez Shin Etsu) | | | | | |

Les résultats obtenus montrent que les dispersions (exemples 3, 16, 17) contenant 5 % de macromonomère siliconé dans le stabilisant et de 5 à 20 % de macromonomère siliconé dans les particules sont stbales tandis que la dispersion de l'exemple 18 contenant 10 % de macromonomère siliconé dans le stabilisant et 10 % de macromonomère siliconé dans les particules n'est pas stable.

### Etude de compatibilité avec le propulseur isobutane :

On a préparé les compositions suivantes avec les dispersions huileuses des exemples 1, 3 et 4 :

| | |
|---|---|
| Dispersion huileuse | 8 % MA |
| Huile PDMS 10 cst | 29 % |
| Isododécane | 63 % |

On a introduit dans un flacon aérosol en verre, 4,45 g de chaque composition, puis serti le flacon avec une valve de distribution, puis on a introduit dans le bidon 25,25 g d'isobutane.

On a observé l'aspect (stabilité) de la composition ainsi conditionnée.

On a obtenu les résultats suivants :

| Exemple dispersion huileuse | 1 | 3 | 4 |
|---|---|---|---|
| Stabilité | Stable | Stable | stable |

Les résultats obtenus montrent que les dispersions huileuses des exemples 1, 3 et 4 sont compatibles avec l'isobutane, en présence d'huile PDMS. Elles conviennet donc à la formulation de compositions aérosol.

### Exemple 19 :

On prépare une composition de maquillage de la peau comprenant les ingrédients suivants :

| | |
|---|---|
| Dispersion de polymère de l'exemple 1 | 85 % |
| dodecamethylpentasiloxane | 10 % |
| Oxydes de fer | 5 % |

La composition appliquée sur la peau permet d'obtenir un film de maquillage brillant, résistant aux huiles et non collant.

La dispersion de polymère de l'exemple 1 peut être remplacée par les dispersions des exemples 2 à 7.

### Exemple 20 :

On prépare une composition de maquillage des lèvres comprenant les ingrédients suivants :

| | |
|---|---|
| Dispersion de polymère de l'exemple 1 | 84 % |
| Polyphényltriméthylsiloxy diméthylsiloxane | |
| (Belsil® PDM 1000 de chez Wacker) | 15 % |
| Red 7 | 1 % |

La composition appliquée sur les lèvres permet d'obtenir un film de maquillage brillant, résistant aux huiles et non collant.

La dispersion de polymère de l'exemple 1 peut être remplacée par les dispersions des exemples 2 à 7.

### Exemple 21 :

On prépare une composition de maquillage des cils comprenant les ingrédients suivants :

| | |
|---|---|
| Dispersion de polymère de l'exemple 1 | 60 % |
| isododécane | 20 % |
| oxydes de fer noir | 20 % |

La composition appliquée sur les cils permet d'obtenir un film de maquillage brillant, résistant aux huiles et non collant.

La dispersion de polymère de l'exemple 1 peut être remplacée par les dispersions des exemples 2 à 7.

### Exemple 22 :

On prépare une composition aérosol filmogène pour la peau comprenant les ingrédients suivants (conditionnée dans un bidon aréosol muni d'une valve de distribution :

| | |
|---|---|
| Dispersion de polymère de l'exemple 1 | 1,2 % MA |
| PDMS 10 cst | 4,35 % |
| Isododécane | 9,45 % |
| Isobutane | qsp 100 % |

La composition est vaporiése sur la peau et forme après séchage un film.

## Revendications

1. Dispersion de particules d'au moins un polymère stabilisées par un agent stabilisant dans un milieu non aqueux contenant au moins une huile hydrocarbonée, le polymère des particules étant un polymère de (méth)acrylate d'alkyle en C₁-C₄ et éventuellement d'un macromonomère siliconé (I) : 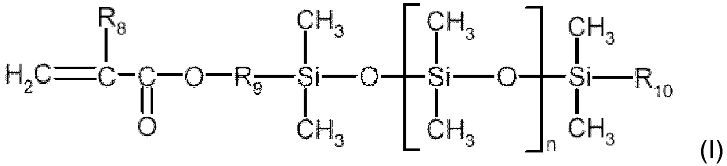 dans laquelle :
- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone, de préférence ayant de 2 à 4 atomes de carbone, et contenant éventuellement une ou deux liaisons éther -O- ; de préférence un groupe éthylène, propylène ou butylène;
- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100 ;
; l'agent stabilisant étant un polymère comprenant de 50 à 100 % en poids, par rapport au poids total du stabilisant, d'acrylate d'alkyle en C₈-C₂₂, de 0 à 50 % en poids de (méth)acrylate d'alkyle en C₁-C₄ et éventuellement de macronomère siliconé (I) :
le stabilisant et/ou le polymère des particules comprenant au moins le macromonomère siliconé (I), et :
(i) lorsque le stabilisant comprend le macromonomère siliconé (I), le macromonomère est présent dans le stabilisant en une teneur inférieure ou égale à 5,5 % en poids, par rapport au poids total de l'ensemble stabilisant + polymère des particules ; le polymère des particules comprenant éventuellement ledit macromonomère siliconé (I) en une teneur inférieure ou égale à 28 % en poids, par rapport au poids total de l'ensemble stabilisant + polymère des particules ;
(ii) lorsque le polymère des particules comprend le macromonomère siliconé (I) et que le stabilisant ne comprend pas de macromonomère siliconé (I), le macromonomère est présent en une teneur inférieure ou égale à 18 % en poids, par rapport au poids total de l'ensemble stabilisant + polymère des particules.

2. Dispersion selon la revendication 1, **caractérisée en ce que** le polymère des particules est un polymère d'acrylate de méthyle et/ou d'acrylate d'éthyle.

3. Dispersion selon l'une des revendications précédentes, **caractérisée en ce que** le polymère des particules comprend un monomère acide à insaturation éthylénique ou leur anhydride, de préférence choisi parmi l'acide (méth)acrylique, l'acide maléique et l'anhydride maléique.

4. Dispersion selon l'une des revendications précédentes, **caractérisée en ce que** le polymère des particules comprend de 62 à 100 % en poids, de (méth)acrylate d'alkyle en C₁-C₄ , de 0 à 38 % en poids du macromonomère siliconé (I) et de 0 à 20 % en poids de monomère acide à insaturation éthylénique, par rapport au poids total du polymère.

5. Dispersion selon l'une des revendications précédentes, **caractérisée en ce que** le polymère des particules est choisi parmi :
les homopolymères d'acrylate de méthyle
les copolymères acrylate de méthyle/macromonomère siliconé (I)
les homopolymères d'acrylate d'éthyle
les copolymères acrylate d'éthyle/macromonomère siliconé (I)
les copolymères acrylate de méthyle/acrylate d'éthyle
les copolymères acrylate de méthyle/acrylate d'éthyle/macromonomère siliconé (I)
les copolymères acrylate de méthyle/acrylate d'éthyle/acide acrylique
les copolymères acrylate de méthyle/acrylate d'éthyle/acide acrylique/macromonomère siliconé (I)
les copolymères acrylate de méthyle/acrylate d'éthyle/anhydride maléique
les copolymères acrylate de méthyle/acrylate d'éthyle/anhydride maléique/macromonomère siliconé (I)
les copolymères acrylate de méthyle/acide acrylique
les copolymères acrylate de méthyle/acide acrylique/macromonomère siliconé (I)
les copolymères acrylate d'éthyle/acide acrylique
les copolymères acrylate d'éthyle/acide acrylique/macromonomère siliconé (I)
les copolymères acrylate de méthyle/anhydride maléique
les copolymères acrylate de méthyle/anhydride maléique/macromonomère siliconé (I)
les copolymères acrylate d'éthyle/anhydride maléique/macromonomère siliconé (I)
les copolymères acrylate d'éthyle/anhydride maléique ;
et de préférence parmi :
les copolymères acrylate de méthyle/acrylate
les copolymères acrylate de méthyle/acrylate d'éthyle/macromonomère siliconé (I)
les copolymères acrylate de méthyle/acrylate d'éthyle/acide acrylique

6. Dispersion selon l'une des revendications précédentes, **caractérisée en ce que** le polymère des particules est présent en une teneur allant de 20 % à 60 % en poids, par rapport au poids total de la dispersion.

7. Dispersion selon l'une des revendications précédentes, **caractérisée en ce que** les particules de polymère ont une taille moyenne allant de 50 à 500 nm, notamment allant de 75 à 400 nm, et mieux allant de 100 à 250 nm.

8. Dispersion selon l'une des revendications précédentes, **caractérisée en ce que** l'agent stabilisant est un polymère comprenant de 50 à 100 % en poids, de préférence de 60 à 95 % en poids, d'acrylate d'alkyle en C₈-C₂₂, de 0 à 50 % en poids, de préférence de 5 à 40 % en poids, de (méth)acrylate d'alkyle en C₁-C₄ et éventuellement le macronomère siliconé (I).

9. Dispersion selon l'une des revendications précédentes, **caractérisée en ce que** l'agent stabilisant est un polymère statistique.

10. Dispersion selon l'une des revendications précédentes, **caractérisée en ce que** l'acrylate d'alkyle en C₈-C₂₂ comprend un groupe choisi parmi groupe 2-éthyle hexyle, isobornyle, lauryle, béhényle ou stéaryle ; de préférence est l'acrylate d'isobornyle ou l'acrylate de 2-éthylhexyle ; préférentiellement est l'acrylate d'isobornyle.

11. Dispersion selon l'une des revendications précédentes, **caractérisée en ce que** l'agent stabilisant est choisi parmi :
les homopolymères d'acrylate de 2-éthyl hexyle
les homopolymères d'acrylate d'isobornyle
les copolymères d'acrylate d'isobornyle/acrylate de méthyle
les copolymères d'acrylate 2-éthyl hexyle /acrylate de méthyle
les copolymères d'acrylate d'isobornyle/acrylate de méthyle/acrylate d'éthyle
les copolymères d'acrylate de 2-éthyl hexyle/acrylate de méthyle/acrylate d'éthyle
les copolymères d'acrylate d'isobornyle/acrylate de méthyle/acrylate d'éthyle/ macromonomère siliconé (I)
les copolymères d'acrylate de 2-éthylhexyle/acrylate de méthyle/acrylate d'éthyle/ macromonomère siliconé (I).

12. Dispersion selon l'une des revendications précédentes, **caractérisée en ce que** le macromonomère siliconé (I) est présent dans le stabilisant en une teneur inférieure ou égale à 5,5 % en poids, notamment allant de 0,1 à 5,5 % en poids, par rapport au poids total de l'ensemble stabilisant + polymère des particules ; et le macromonomère siliconé (I) n'est pas présent dans le polymère des particules.

13. Dispersion selon la revendication précédente, **caractérisée en ce que** l'ensemble stabilisant + polymère des particules présents dans la dispersion comprend de 5 à 50 % en poids d'acrylate d'alkyle en C₈-C₂₂ polymérisé, de 44,5 à 89,5 % en poids de (méth)acrylate d'alkyle en C₁-C₄ polymérisé et de 0,1 à 5,5 % en poids de macromonomère siliconé (I), par rapport au poids total de l'ensemble stabilisant + polymère des particules ;
Préférentiellement, l'ensemble stabilisant + polymère des particules présent dans la dispersion comprend de 6 à 30 % en poids d'acrylate d'alkyle en C₈-C₂₂ polymérisé et de 64,5 à 93,9 % en poids de (méth)acrylate d'alkyle en C₁-C₄ polymérisé et de 0,1 à 5,5 % en poids de macromonomère siliconé (I), par rapport au poids total de l'ensemble stabilisant + polymère des particules.

14. Dispersion selon l'une des revendications 1 à 11, **caractérisée en ce que** le macromonomère siliconé (I) est présent dans le stabilisant en une teneur inférieure ou égale à 5,5 % en poids, notamment allant de 0,1 à 5,5 % en poids, par rapport au poids total de l'ensemble stabilisant + polymère des particules ; et il est présent dans le polymère des particules en une teneur inférieure ou égale à 28 % en poids, notamment allant de 0,1 à 28 % en poids, par rapport au poids total de l'ensemble stabilisant + polymère des particules.

15. Dispersion selon la revendication précédente, **caractérisée en ce que** l'ensemble stabilisant + polymère des particules présents dans la dispersion comprend de 5 à 50 % en poids d'acrylate d'alkyle en C₈-C₂₂ polymérisé, de 16,5 à 94,9 % en poids de (méth)acrylate d'alkyle en C₁-C₄ polymérisé et de 0,1 à 33,5 % en poids de macromonomère siliconé (I), par rapport au poids total de l'ensemble stabilisant + polymère des particules ;
Préférentiellement, l'ensemble stabilisant + polymère des particules présent dans la dispersion comprend de 6 à 30 % en poids d'acrylate d'alkyle en C₈-C₂₂ polymérisé et de 36,5 à 93,9 % en poids de (méth)acrylate d'alkyle en C₁-C₄ polymérisé et de 0,1 à 33,5 % en poids de macromonomère siliconé (I), par rapport au poids total de l'ensemble stabilisant + polymère des particules.

16. Dispersion selon l'une des revendications 1 à 11, **caractérisée en ce que** le macromonomère siliconé (I) est présent dans le polymère des particules en une teneur inférieure ou égale à 18 % en poids, notamment allant de 0,1 à 18 % en poids, par rapport au poids total de l'ensemble stabilisant + polymère des particules ; et il n'est pas présent dans le stabilisant.

17. Dispersion selon la revendication précédente, **caractérisée en ce que** l'ensemble stabilisant + polymère des particules présents dans la dispersion comprend de 5 à 50 % en poids d'acrylate d'alkyle en C₈-C₂₂ polymérisé , de 32 à 94,9 % en poids de (méth)acrylate d'alkyle en C₁-C₄ polymérisé et de 0,1 à 18 % en poids de macromonomère siliconé (I), par rapport au poids total de l'ensemble stabilisant + polymère des particules ;
Préférentiellement, l'ensemble stabilisant + polymère des particules présent dans la dispersion comprend de 6 à 30 % en poids d'acrylate d'alkyle en C₈-C₂₂ polymérisé et de 52 à 93,9 % en poids de (méth)acrylate d'alkyle en C₁-C₄ polymérisé et de 0,1 à 18 % en poids de macromonomère siliconé (I), par rapport au poids total de l'ensemble stabilisant + polymère des particules.

18. Dispersion selon l'une des revendications précédentes, **caractérisée en ce que** l'huile hydrocarbonée est choisie parmi les huiles hydrocarbonées, de préférence apolaires, ayant de 8 à 16 atomes de carbone.

19. Dispersion selon l'une des revendications précédentes, **caractérisée en ce que** l'huile hydrocarbonée est l'isododécane.

20. Dispersion selon l'une des revendications précédentes, **caractérisée** en ce le polymère des particules est présent dans la dispersion en une teneur allant de 20 % à 60 % en poids, par rapport au poids total de la dispersion.

21. Composition comprenant, dans un milieu physiologiquement acceptable, une dispersion de polymère selon l'une des revendications précédentes.

22. Composition selon la revendication précédente, **caractérisée en ce qu'**elle comprend un additif cosmétique choisi parmi l'eau, les parfums, les conservateurs, les charges, les matières colorantes, les filtres UV, les huiles, les cires, les tensioactifs, les hydratants, les vitamines, les céramides, les antioxydants, les agents anti radicaux libres, les polymères, les épaississants.

23. composition selon l'une des revendications 21 ou 22, **caractérisée en ce qu'**elle comprend une huile siliconée, notamment choisie parmi les huiles de silicones linéaires ou cycliques volatiles, ayant notamment de 2 à 10 atomes de silicium, de préférence de 2 à 7 atomes de silicium, ces silicones comportant éventuellement des groupes alkyle ou alkoxy ayant de 1 à 10 atomes de carbone, comme par exemple l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane ;
les polydiméthylsiloxanes (PDMS) comportant des groupements alkyle ou alcoxy pendants et/ou en bouts de chaîne siliconée, groupements ayant chacun de 2 à 60 atomes de carbone, notamment les alkyl (C₂-C₆₀) dimethicones ; les alkyl(C₂-C₆₀) methicones ; les silicones phénylées comme les phényl triméthicones, les phényl diméthicones, les phényl triméthylsiloxy diphénylsiloxanes, les diphényl diméthicones, les triméthylsiloxyphenyl diméthicone, les diphényl méthyldiphényl trisiloxanes ;
et leurs mélanges.

24. Composition selon la revendication précédente, **caractérisée en ce que** l'huile siliconée est présente en une teneur allant de 0,1 à 60 % en poids, par rapport au poids total de la composition, de préférence allant de 1 à 50 % en poids, et mieux allant de 1 à 40 % en poids.

25. Composition selon l'une des revendications 21 à 24, **caractérisée en ce qu'**elle est une comtion aérosol contenant un agent propulseur, notamment choisi parmi le diméthyléther, les hydrocarbures chlorés et/ou fluorés tels que le trichlorofluorométhane, le dichlorodifluorométhane, le chlorodifluorométhane, le 1,1,1,2-tétrafluoroéthane, le chloropentafluoroéthane, le 1-chloro-1,1-difluoroéthane, le 1,1-difluoroéthane, ou des hydrocarbures volatils, tels que notamment les alcanes en C₃₋₅, comme le propane, l'isopropane, le n-butane, l'isobutane, le pentane, ou des gaz comprimés comme l'air, l'azote, le gaz carbonique, et leurs mélanges ; de préférence choisi parmi le diméthyléther, le 1,1,1,2-tétrafluoroéthane, et les alcanes en C₃₋₅ et en particulier le propane, le n-butane, l'isobutane et leurs mélanges ; préférentiellement l'isobutane.

26. Procédé cosmétique non thérapeutique de traitement des matières kératiniques, comprenant l'application sur les matières kératiniques d'une composition selon l'une des revendications 21 à 25, en particulier pour le soin ou le maquillage des matières kératiniques.

## Patentansprüche

1. Dispersion von durch mindestens einen Stabilisator stabilisierten Teilchen mindestens eines Polymers, in einem nicht wässrigen Medium, das mindestens ein Kohlenwasserstofföl umfasst, wobei es sich bei dem Polymer der Teilchen um ein Polymer von C₁-C₄-Alkyl(meth)acrylat und gegebenenfalls einem Silikon-Makromonomer (I) handelt: 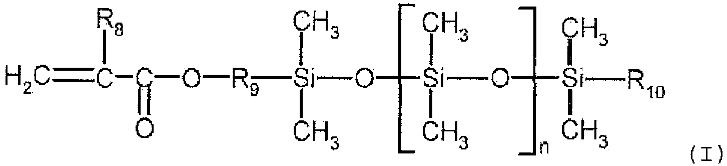 in dem:
- R8 für ein Wasserstoffatom oder eine Methylgruppe, bevorzugt Methyl, steht;
- R9 für eine lineare oder verzweigte, bevorzugt lineare, zweiwertige Kohlenwasserstoffgruppe mit 1 bis 10 Kohlenstoffatomen, bevorzugt mit 2 bis 4 Kohlenstoffatomen, und gegebenenfalls mit einer oder zwei Etherbindungen -O-, bevorzugt eine Ethylen-, Propylen- oder Butylengruppe, steht;
- R10 für eine lineare oder verzweigte Alkylgruppe mit 1 bis 10 Kohlenstoffatomen, insbesondere 2 bis 8 Kohlenstoffatomen, bevorzugt Methyl, Ethyl, Propyl, Butyl oder Pentyl, steht;
- n für eine ganze Zahl im Bereich von 1 bis 300, bevorzugt im Bereich von 3 bis 200, bevorzugt im Bereich von 5 bis 100, steht;
wobei es sich bei dem Stabilisator um ein Polymer handelt, das 50 bis 100 Gew.-%, bezogen auf das Gesamtgewicht des Stabilisators, C₈-C₂₂-Alkylacrylat, 0 bis 50 Gew.-% C₁-C₄-Alkyl(meth)acrylat und gegebenenfalls Silikon-Makromonomer (I) umfasst;
wobei der Stabilisator und/oder das Polymer der Teilchen mindestens das Silikon-Makromonomer (I) umfassen/umfasst und
(i) dann, wenn der Stabilisator das Silikon-Makromonomer (I) umfasst, das Makromonomer in dem Stabilisator in einem Gehalt kleiner oder gleich 5,5 Gew.-%, bezogen auf das Gesamtgewicht der Summe von Stabilisator + Polymer der Teilchen, vorliegt, wobei das Polymer der Teilchen gegebenenfalls das Silikon-Makromonomer (I) in einem Gehalt kleiner oder gleich 28 Gew.-%, bezogen auf das Gesamtgewicht der Summe von Stabilisator + Polymer der Teilchen, umfasst;
(ii) dann, wenn das Polymer der Teilchen das Silikon-Makromonomer (I) umfasst und der Stabilisator kein Silikon-Makromonomer (I) umfasst, das Makromonomer in einem Gehalt kleiner oder gleich 18 Gew.-%, bezogen auf das Gesamtgewicht der Summe von Stabilisator + Polymer der Teilchen, vorliegt.

2. Dispersion nach Anspruch 1, **dadurch gekennzeichnet, dass** es sich bei dem Polymer der Teilchen um ein Methylacrylat- und/oder Ethylacrylat-Polymer handelt.

3. Dispersion nach einem der vorhergehenden Ansprüche, **dadurch gekennzeichnet, dass** das Polymer der Teilchen ein ethylenisch ungesättigtes Säuremonomer oder Anhydrid davon, das vorzugsweise aus (Meth)acrylsäure, Maleinsäure und Maleinsäureanhydrid ausgewählt ist, umfasst.

4. Dispersion nach einem der vorhergehenden Ansprüche, **dadurch gekennzeichnet, dass** das Polymer der Teilchen 62 bis 100 Gew.-% C₁-C₄-Alkyl-(meth)acrylat, 0 bis 38 Gew.-% des Silikon-Makromonomers (I) und 0 bis 20 Gew.-% ethylenisch ungesättigtes Säuremonomer, bezogen auf das Gesamtgewicht des Polymers, umfasst.

5. Dispersion nach einem der vorhergehenden Ansprüche, **dadurch gekennzeichnet, dass** das Polymer der Teilchen ausgewählt ist aus:
Methylacrylat-Homopolymeren
Methylacrylat/Silikon-Makromonomer-(I)-Copolymeren
Ethylacrylat-Homopolymeren
Ethylacrylat/Silikon-Makromonomer-(I)-Copolymeren
Methylacrylat/Ethylacrylat-Copolymeren
Methylacrylat/Ethylacrylat/Silikon-Makromonomer-(I)-Copolymeren
Methylacrylat/Ethylacrylat/Acrylsäure-Copolymeren
Methylacrylat/Ethylacrylat/Acrylsäure/Silikon-Makromonomer-(I)-Copolymeren
Methylacrylat/Ethylacrylat/Maleinsäureanhydrid-Copolymeren
Methylacrylat/Ethylacrylat/Maleinsäureanhydrid/Silikon-Makromonomer- (I) -Copolymeren
Methylacrylat/Acrylsäure-Copolymeren
Methylacrylat/Acrylsäure/Silikon-Makromonomer-(I)-Copolymeren
Ethylacrylat/Acrylsäure-Copolymeren
Ethylacrylat/Acrylsäure/Silikon-Makromonomer-(I)-Copolymeren
Methylacrylat/Maleinsäureanhydrid-Copolymeren
Methylacrylat/Maleinsäureanhydrid/Silikon-Makromonomer-(I)-Copolymeren
Ethylacrylat/Maleinsäureanhydrid/Silikon-Makromonomer-(I)-Copolymeren
Ethylacrylat/Maleinsäureanhydrid-Copolymeren und bevorzugt aus:
Methylacrylat/Acrylat-Copolymeren
Methylacrylat/Ethylacrylat/Silikon-Makromonomer-(I)-Copolymeren
Methylacrylat/Ethylacrylat/Acrylsäure-Copolymeren.

6. Dispersion nach einem der vorhergehenden Ansprüche, **dadurch gekennzeichnet, dass** das Polymer der Teilchen in einem Gehalt im Bereich von 20 bis 60 Gew.-%, bezogen auf das Gesamtgewicht der Dispersion, vorliegt.

7. Dispersion nach einem der vorhergehenden Ansprüche, **dadurch gekennzeichnet, dass** die Polymerteilchen der Teilchen eine mittlere Größe im Bereich von 50 bis 500 nm, insbesondere im Bereich von 75 bis 400 nm und noch besser im Bereich von 100 bis 250 nm aufweisen.

8. Dispersion nach einem der vorhergehenden Ansprüche, **dadurch gekennzeichnet, dass** es sich bei dem Stabilisator um ein Polymer handelt, das 50 bis 100 Gew.-%, bevorzugt 60 bis 95 Gew.-%, C₈-C₂₂-Alkylacrylat, 0 bis 50 Gew.-%, bevorzugt 5 bis 40 Gew.-%, C₁-C₄-Alkyl (meth) acrylat und gegebenenfalls das Silikon-Makromonomer (I) umfasst.

9. Dispersion nach einem der vorhergehenden Ansprüche, **dadurch gekennzeichnet, dass** es sich bei dem Stabilisator um ein statistisches Polymer handelt.

10. Dispersion nach einem der vorhergehenden Ansprüche, **dadurch gekennzeichnet, dass** das C₈-C₂₂-Alkylacrylat eine Gruppe, die aus der Gruppe 2-Ethylhexyl, Isobornyl, Lauryl, Behenyl oder Stearyl ausgewählt ist, umfasst, bevorzugt Isobornylacrylat oder 2-Ethylhexylacrylat ist und vorzugsweise Isobornylacrylat ist.

11. Dispersion nach einem der vorhergehenden Ansprüche, **dadurch gekennzeichnet, dass** der Stabilisator ausgewählt ist aus:
2-Ethylhexylacrylat-Homöpolymeren
Isobornylacrylat-Homopolymeren
Isöbornylacrylat/Methylacrylat-Copolymeren
2-Ethylhexylacrylat/Methylacrylat-Copolymeren
Isobornylacrylat/Methylacrylat/Ethylacrylat-Copolymeren
2-Ethylhexylacrylät/Methylacrylat/Ethylacrylat-Copolymeren
Isobornylacrylat/
Methylacrylat/Ethylacrylat/Silikon-Makromonomer-(I)-Copolymeren
2-Ethylhexylacrylat/Methylacrylat/Ethylacrylat/ Silikon-Makromonomer-(I)-Copolymeren.

12. Dispersion nach einem der vorhergehenden Ansprüche, **dadurch gekennzeichnet, dass** das Silikon-Makromonomer (I) in dem Stabilisator in einem Gehalt kleiner oder gleich 5,5 Gew.-%, insbesondere im Bereich von 0,1 bis 5,5 Gew.-%, bezogen auf das Gesamtgewicht der Summe von Stabilisator + Polymer der Teilchen, vorliegt und das Silikon-Makromonomer (I) nicht in dem Polymer der Teilchen vorliegt.

13. Dispersion nach dem vorhergehenden Anspruch, **dadurch gekennzeichnet, dass** die Summe von Stabilisator + Polymer der Teilchen, die in der Dispersion vorliegen, 5 bis 50 Gew.-% einpolymerisiertes C₈-C₂₂-Alkylacrylat, 44,5 bis 89,5 Gew.-% einpolymerisiertes C₁-C₄-Alkyl(meth)acrylat und 0,1 bis 5,5 Gew.-% Silikon-Makromonomer (I), bezogen auf das Gesamtgewicht der Summe von Stabilisator + Polymer der Teilchen, umfasst, vorzugsweise die Summe von Stabilisator + Polymer der Teilchen, die in der Dispersion vorliegen, 6 bis 3 0 Gew.-% einpolymerisiertes C₈-C₂₂-Alkylacrylat, 64,5 bis 93,9 Gew.-% einpolymerisiertes C₁-C₄-Alkyl(meth)acrylat und 0,1 bis 5,5 Gew.-% Silikon-Makromonomer (I), bezogen auf das Gesamtgewicht der Summe von Stabilisator + Polymer der Teilchen, umfasst.

14. Dispersion nach einem der Ansprüche 1 bis 11, **dadurch gekennzeichnet, dass** das Silikon-Makromonomer (I) in dem Stabilisator in einem Gehalt kleiner oder gleich 5,5 Gew.-%, insbesondere im Bereich von 0,1 bis 5,5 Gew.-%, bezogen auf das Gesamtgewicht der Summe von Stabilisator + Polymer der Teilchen, vorliegt und es in dem Polymer der Teilchen in einem Gehalt kleiner oder gleich 28 Gew.-%, insbesondere im Bereich von 0,1 bis 28 Gew.-%, bezogen auf das Gesamtgewicht der Summe von Stabilisator + Polymer der Teilchen, vorliegt.

15. Dispersion nach dem vorhergehenden Anspruch, **dadurch gekennzeichnet, dass** die Summe von Stabilisator + Polymer der Teilchen, die in der Dispersion vorliegen, 5 bis 50 Gew.-% einpolymerisiertes C₈-C₂₂-Alkylacrylat, 16,5 bis 94,9 Gew.-% einpolymerisiertes C₁-C₄-Alkyl(meth)acrylat und 0,1 bis 33,5 Gew.-% Silikon-Makromonomer (I), bezogen auf das Gesamtgewicht der Summe von Stabilisator + Polymer der Teilchen, umfasst, vorzugsweise die Summe von Stabilisator + Polymer der Teilchen, die in der Dispersion vorliegen, 6 bis 30 Gew.-% einpolymerisiertes C₈-C₂₂-Alkylacrylat, 36,5 bis 93,9 Gew.-% einpolymerisiertes C₁-C₄-Alkyl (meth) acrylat und 0,1 bis 33,5 Gew.-% Silikon-Makromonomer (I), bezogen auf das Gesamtgewicht der Summe von Stabilisator + Polymer der Teilchen, umfasst.

16. Dispersion nach einem der Ansprüche 1 bis 11, **dadurch gekennzeichnet, dass** das Silikon-Makromonomer (I) in dem Polymer der Teilchen in einem Gehalt kleiner oder gleich 18 Gew.-%, insbesondere im Bereich von 0,1 bis 18 Gew.-%, bezogen auf das Gesamtgewicht der Summe von Stabilisator + Polymer der Teilchen vorliegt und in dem Stabilisator nicht vorliegt.

17. Dispersion nach dem vorhergehenden Anspruch, **dadurch gekennzeichnet, dass** die Summe von Stabilisator + Polymer der Teilchen, die in der Dispersion vorliegen, 5 bis 50 Gew.-% einpolymerisiertes C₈-C₂₂-Alkylacrylat, 32 bis 94,9 Gew.-% einpolymerisiertes C₁-C₄-Alkyl(meth)acrylat und 0,1 bis 18 Gew.-% Silikon-Makromonomer (I), bezogen auf das Gesamtgewicht der Summe von Stabilisator + Polymer der Teilchen, umfasst, vorzugsweise die Summe von Stabilisator + Polymer der Teilchen, die in der Dispersion vorliegen, 6 bis 30 Gew.-% einpolymerisiertes C₈-C₂₂-Alkylacrylat, 52 bis 93,9 Gew.-% einpolymerisiertes C₁-C₄-Alkyl(meth)acrylat und 0,1 bis 18 Gew.-% Silikon-Makromonomer (I), bezogen auf das Gesamtgewicht der Summe von Stabilisator + Polymer der Teilchen, umfasst.

18. Dispersion nach einem der vorhergehenden Ansprüche, **dadurch gekennzeichnet, dass** das Kohlenwasserstofföl aus vorzugsweise unpolaren Kohlenwasserstoffölen mit 8 bis 16 Kohlenstoffatomen ausgewählt ist.

19. Dispersion nach einem der vorhergehenden Ansprüche, **dadurch gekennzeichnet, dass** es sich bei dem Kohlenwasserstofföl um Isododecan handelt.

20. Dispersion nach einem der vorhergehenden Ansprüche, **dadurch gekennzeichnet, dass** das Polymer der Teilchen in der Dispersion in einem Gehalt im Bereich von 20 bis 60 Gew.-%, bezogen auf das Gesamtgewicht der Dispersion, vorliegt.

21. Zusammensetzung, die in einem physiologisch unbedenklichen Medium eine Polymerdispersion nach einem der vorhergehenden Ansprüche umfasst.

22. Zusammensetzung nach dem vorhergehenden Anspruch, **dadurch gekennzeichnet, dass** sie ein kosmetisches Additiv umfasst, das aus Wasser, Duftstoffen, Konservierungsstoffen, Füllstoffen, Farbmitteln, UV-Filtern, Ölen, Wachsen, Tensiden, Feuchtigkeitsmitteln, Vitaminen, Ceramiden, Antioxidantien, Mitteln gegen freie Radikale, Polymeren und Verdickungsmitteln ausgewählt ist.

23. Zusammensetzung nach einem der Ansprüche 21 oder 22, **dadurch gekennzeichnet, dass** sie ein Silikonöl umfasst, das insbesondere ausgewählt ist aus linearen oder cyclischen flüchtigen Silikonölen mit insbesondere 2 bis 10 Siliciumatomen und vorzugsweise 2 bis 7 Siliciumatomen, wobei diese Silikone gegebenenfalls Alkyl- oder Alkoxygruppen mit 1 bis 10 Kohlenstoffatomen umfassen, wie beispielsweise Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, octamethyltrisiloxan, Decamethyltetrasiloxan oder Dodecamethylpentasiloxan;
Polydimethylsiloxanen (PDMS) mit seitenständigen und/oder an den Enden der Silikonkette angeordneten Alkyl- oder Alkoxygruppen, Gruppen mit jeweils 2 bis 60 Kohlenstoffatomen, insbesondere (C₂-C₆₀) Alkyldimethiconen; (C₂-C₆₀) Alkylmethiconen; Phenylsilikonen, beispielsweise Phenyltrimethiconen, Phenyldimethiconen, Phenyltrimethylsiloxydiphenylsiloxanen, Diphenyldimethiconen, Trimethylsiloxyphenyldimethicon und Diphenylmethyldiphenyltrisiloxanen;
und Mischungen davon.

24. Zusammensetzung nach dem vorhergehenden Anspruch, **dadurch gekennzeichnet, dass** das Silikonöl in einem Gehalt im Bereich von 0,1 bis 60 Gew.-%, bezogen auf das Gesamtgewicht der Zusammensetzung, vorzugsweise im Bereich von 1 bis 50 Gew.-% und noch besser im Bereich von 1 bis 40 Gew.-% vorliegt.

25. Zusammensetzung nach einem der Ansprüche 21 bis 24, **dadurch gekennzeichnet, dass** es sich um eine Aerosolzusammensetzung handelt, die ein Treibmittel enthält, das insbesondere aus Dimethylether, chlorierten und/oder fluorierten Kohlenwasserstoffen wie Trichlorfluormethan, Dichlordifluormethan, Chlordifluormethan, 1,1,1,2-Tetrafluorethan, Chlorpentafluörethan, 1-Chlor-1,1-difluorethan und 1,1-Difluorethan oder flüchtigen Kohlenwasserstoffen wie insbesondere C₃₋₅-Alkanen wie Propan, Isopropan, n-Butan, Isobutan und Pentan oder Druckgasen wie Luft, Stickstoff, Kohlensäure und Mischungen davon ausgewählt ist und bevorzugt aus Dimethylether, 1,1,1,2-Tetrafluorethan und C₃₋₅-Alkanen und insbesondere Propan, n-Butan, Isobutan und Mischungen davon und vorzugsweise Isobutan ausgewählt ist.

26. Nichttherapeutisches Verfahren zur Behandlung von Keratinmaterialien, bei dem man auf die Keratinmaterialien eine Zusammensetzung nach einem der Ansprüche 21 bis 25 aufbringt, insbesondere zur Pflege oder zum Make-up von Keratinmaterialien.

## Claims

1. Dispersion of particles of at least one polymer stabilized with a stabilizer in a nonaqueous medium containing at least one hydrocarbon-based oil, the polymer of the particles being a polymer of C₁-C₄ alkyl (meth)acrylate and optionally of a silicone macromonomer (I): 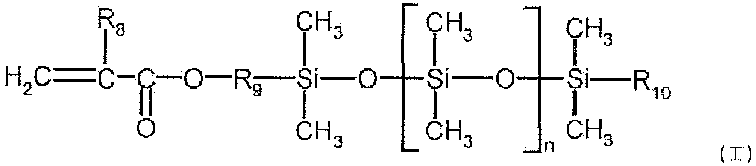 in which:
- R8 denotes a hydrogen atom or a methyl group; preferably methyl;
- R9 denotes a linear or branched, preferably linear, divalent hydrocarbon-based group containing from 1 to 10 carbon atoms, preferably containing from 2 to 4 carbon atoms, and optionally containing one or two -O- ether bonds; preferably an ethylene, propylene or butylene group;
- R10 denotes a linear or branched alkyl group containing from 1 to 10 carbon atoms, especially from 2 to 8 carbon atoms; preferably methyl, ethyl, propyl, butyl or pentyl;
- n denotes an integer ranging from 1 to 300, preferably ranging from 3 to 200 and preferentially ranging from 5 to 100;
the stabilizer being a polymer comprising from 50% to 100% by weight, relative to the total weight of the stabilizer, of C₈-C₂₂ alkyl acrylate, from 0 to 50% by weight of C₁-C₄ alkyl (meth) acrylate and optionally silicone macromonomer (I):
the stabilizer and/or the polymer of the particles comprising at least the silicone macromonomer (I), and:
(i) when the stabilizer comprises the silicone macromonomer (I), the macromonomer is present in the stabilizer in a content of less than or equal to 5.5% by weight, relative to the total weight of the combination of stabilizer + polymer of the particles; the polymer of the particles optionally comprising said silicone macromonomer (I) in a content of less than or equal to 28% by weight, relative to the total weight of the combination of stabilizer + polymer of the particles;
(ii) when the polymer of the particles comprises the silicone macromonomer (I) and the stabilizer does not comprise silicone macromonomer (I), the macromonomer is present in a content of less than or equal to 18% by weight, relative to the total weight of the combination of stabilizer + polymer of the particles.

2. Dispersion according to Claim 1, **characterized in that** the polymer of the particles is a methyl acrylate and/or ethyl acrylate polymer.

3. Dispersion according to either of the preceding claims, **characterized in that** the polymer of the particles comprises an ethylenically unsaturated acid monomer Or the anhydride thereof, preferably chosen from (meth)acrylic acid, maleic acid and maleic anhydride.

4. Dispersion according to one of the preceding claims, **characterized in that** the polymer of the particles comprises from 62% to 100% by weight of C₁-C₄ alkyl (meth)acrylate, from 0 to 38% by weight of the silicone macromonomer (I) and from 0 to 20% by weight of ethylenically unsaturated acid monomer, relative to the total weight of the polymer.

5. Dispersion according to one of the preceding claims, **characterized in that** the polymer of the particles is chosen from:
methyl acrylate homopolymers
methyl acrylate/silicone macromonomer (I) copolymers
ethyl acrylate homopolymers
ethyl acrylate/silicone macromonomer (I) copolymers
methyl acrylate/ethyl acrylate copolymers
methyl acrylate/ethyl acrylate/silicone macromonomer (I) copolymers
methyl acrylate/ethyl acrylate/acrylic acid copolymers
methyl acrylate/ethyl acrylate/acrylic acid/silicone macromonomer (I) copolymers
methyl acrylate/ethyl acrylate/maleic anhydride copolymers
methyl acrylate/ethyl acrylate/maleic anhydride/silicone macromonomer (I) copolymers
methyl acrylate/acrylic acid copolymers
methyl acrylate/acrylic acid/silicone macromonomer (I) copolymers
ethyl acrylate/acrylic acid copolymers
ethyl acrylate/acrylic acid/silicone macromonomer (I) copolymers
methyl acrylate/maleic anhydride copolymers
methyl acrylate/maleic anhydride/silicone macromonomer (I) copolymers
ethyl acrylate/maleic anhydride/silicone macromonomer (I) copolymers
ethyl acrylate/maleic anhydride copolymers;
and preferably from:
methyl acrylate/acrylate copolymers
methyl acrylate/ethyl acrylate/silicone macromonomer (I) copolymers
methyl acrylate/ethyl acrylate/acrylic acid copolymers.

6. Dispersion according to one of the preceding claims, **characterized in that** the polymer of the particles is present in a content ranging from 20% to 60% by weight, relative to the total weight of the dispersion.

7. Dispersion according to one of the preceding claims, **Characterized in that** the polymer particles have an average size ranging from 50 to 500 nm, especially ranging from 75 to 400 nm and better still ranging from 100 to 250 nm.

8. Dispersion according to one of the preceding claims, **characterized in that**
the stabilizer is a polymer comprising from 50% to 100% by weight, preferably from 60% to 95% by weight, of C₈-C₂₂ alkyl acrylate, from 0 to 50% by weight, preferably from 5% to 40% by weight, of C₁-C₄ alkyl (meth)acrylate and optionally the silicone macromonomer (I).

9. Dispersion according to one of the preceding claims, **characterized in that** the stabilizer is a statistical polymer.

10. Dispersion according to one of the preceding claims, **characterized in that** the C₈-C₂₂ alkyl acrylate comprises a group chosen from the group 2-ethylhexyl, isobornyl, lauryl, behenyl and stearyl; preferably, it is isobornyl acrylate or 2-ethylhexyl acrylate; preferentially, it is isobornyl acrylate.

11. Dispersion according to one of the preceding claims, **characterized in that** the stabilizer is chosen from:
2-ethylhexyl acrylate homopolymers
isobornyl acrylate homopolymers
isobornyl acrylate/methyl acrylate copolymers
2-ethylhexyl acrylate/methyl acrylate copolymers isobornyl acrylate/methyl acrylate/ethyl acrylate copolymers
2-ethylhexyl acrylate/methyl acrylate/ethyl acrylate copolymers
isobornyl acrylate/methyl acrylate/ethyl acrylate/silicone macromonomer (I) copolymers
2-ethylhexyl acrylate/methyl acrylate/ethyl acrylate/silicone macromonomer (I) copolymers.

12. Dispersion according to one of the preceding claims, **characterized in that** the silicone macromonomer (I) is present in the stabilizer in a content of less than or equal to 5.5% by weight, especially ranging from 0.1% to 5.5% by weight, relative to the total weight of the combination of stabilizer + polymer of the particles; and the silicone macromonomer (I) is not present in the polymer of the particles.

13. Dispersion according to the preceding claim, **characterized in that** the combination of stabilizer + polymer of the particles present in the dispersion comprises from 5% to 50% by weight of polymerized C₈-C₂₂ alkyl acrylate, from 44.5% to 89.5% by weight of polymerized C₁-C₄ alkyl (meth)acrylate and from 0.1% to 5.5% by weight of silicone macromonomer (I), relative to the total weight of the combination of stabilizer + polymer of the particles; preferentially, the combination of stabilizer + polymer of the particles present in the dispersion comprises from 6% to 30% by weight of polymerized C₈-C₂₂ alkyl acrylate, from 64.5% to 93.9% by weight of polymerized C₁-C₄ alkyl (meth)acrylate and from 0.1% to 5.5% by weight of silicone macromonomer (I), relative to the total weight of the combination of stabilizer + polymer of the particles.

14. Dispersion according to one of Claims 1 to 11, **characterized in that** the silicone macromonomer (I) is present in the stabilizer in a content of less than or equal to 5.5% by weight, in particular ranging from 0.1% to 5.5% by weight, relative to the total weight of the combination of stabilizer + polymer of the particles; and it is present in the polymer of the particles in a content of less than or equal to 28% by weight, in particular ranging from 0.1% to 28% by weight, relative to the total weight of the combination of stabilizer + polymer of the particles.

15. Dispersion according to the preceding claim, **characterized in that** the combination of stabilizer + polymer of the particles present in the dispersion comprises from 5% to 50% by weight of polymerized C₈-C₂₂ alkyl acrylate, from 16.5% to 94.9% by weight of polymerized C₁-C₄ alkyl (meth)acrylate and from 0.1% to 33.5% by weight of silicone macromonomer (I), relative to the total weight of the combination of stabilizer + polymer of the particles; preferentially, the combination of stabilizer + polymer of the particles present in the dispersion comprises from 6% to 30% by weight of polymerized C₈-C₂₂ alkyl acrylate, from 36.5% to 93.9% by weight of polymerized C₁-C₄ alkyl (meth)acrylate and from 0.1% to 33.5% by weight of silicone macromonomer (I), relative to the total weight of the combination of stabilizer + polymer of the particles.

16. Dispersion according to one of Claims 1 to 11, **characterized in that** the silicone macromonomer (I) is present in the polymer of the particles in a content of less than or equal to 18% by weight, in particular ranging from 0.1% to 18% by weight, relative to the total weight of the combination of stabilizer + polymer of the particles; and it is not present in the stabilizer.

17. Dispersion according to the preceding claim, **characterized in that** the combination of stabilizer + polymer of the particles present in the dispersion comprises from 5% to 50% by weight of polymerized C₈-C₂₂ alkyl acrylate, from 32% to 94.9% by weight of polymerized C₁-C₄ alkyl (meth)acrylate and from 0.1% to 18% by weight of silicone macromonomer (I), relative to the total weight of the combination of stabilizer + polymer of the particles; preferentially, the combination of stabilizer + polymer of the particles present in the dispersion comprises from 6% to 30% by weight of polymerized C₈-C₂₂ alkyl acrylate, from 52% to 93.9% by weight of polymerized C₁-C₄ alkyl (meth) acrylate and from 0.1% to 18% by weight of silicone macromonomer (I), relative to the total weight of the combination of stabilizer + polymer of the particles.

18. Dispersion according to one of the preceding claims, **characterized in that** the hydrocarbon-based oil is chosen from hydrocarbon-based oils, which are preferably apolar, containing from 8 to 16 carbon atoms.

19. Dispersion according to one of the preceding claims, **characterized in that** the hydrocarbon-based oil is isododecane.

20. Dispersion according to one of the preceding claims, **characterized in that** the polymer of the particles is present in the dispersion in a content ranging from 20% to 60% by weight, relative to the total weight of the dispersion.

21. Composition comprising, in a physiologically acceptable medium, a polymer dispersion according to one of the preceding claims.

22. Composition according to the preceding claim, **characterized in that** it comprises a cosmetic additive chosen from water, fragrances, preserving agents, fillers, dyestuffs, UV-screening agents, oils, waxes, surfactants, moisturizers, vitamins, ceramides, antioxidants, free-radical scavengers, polymers and thickeners.

23. The composition according to either of claims 21 and 22, **characterized in that** it comprises a silicone oil, chosen especially from linear or cyclic volatile silicone oils, especially containing from 2 to 10 silicon atoms, preferably from 2 to 7 silicon atoms, these silicones optionally comprising alkyl or alkoxy groups containing from 1 to 10 carbon atoms, for instance octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane or dodecamethylpentasiloxane;
polydimethylsiloxanes (PDMSs) comprising alkyl or alkoxy groups, that are pendent and/or at the end of a silicone chain, the groups each containing from 2 to 60 carbon atoms, especially (C₂-C₆₀) alkyl dimethicones; (C₂-C₆₀) alkyl methicones; phenyl silicones, for instance phenyl trimethicones, phenyl dimethicones, phenyltrimethylsiloxydiphenylsiloxanes, diphenyl dimethicones, trimethylsiloxyphenyl dimethicone and diphenylmethyldiphenyltrisiloxanes;
and mixtures thereof.

24. The composition according to the preceding claim, **characterized in that** the silicone oil is present in a content ranging from 0.1% to 60% by weight, relative to the total weight of the composition, preferably ranging from 1% to 50% by weight, and better still ranging from 1% to 40% by weight.

25. The composition according to one of claims 21 to 24, **characterized in that** it is an aerosol composition containing a propellant, chosen especially from dimethyl ether, chlorinated and/or fluorinated hydrocarbons such as trichlorofluoromethane, dichlorodifluoromethane, chlorodifluoromethane, 1,1,1,2-tetrafluoroethane, chloropentafluoroethane, 1-chloro-1,1-difluoroethane or 1,1-difluoroethane, or volatile hydrocarbons especially such as C₃₋₅ alkanes, for instance propane, isopropane, n-butane, isobutane or pentane, or compressed gases such as air, nitrogen, carbon dioxide, and mixtures thereof; preferably chosen from dimethyl ether, 1,1,1,2-tetrafluoroethane, and C₃₋₅ alkanes and in particular propane, n-butane and isobutane, and mixtures thereof; preferentially isobutane.

26. A nontherapeutic cosmetic process for treating keratin materials, comprising the application to the keratin materials of a composition according to one of claims 21 to 25, in particular for caring for or making up keratin materials.
